# EUROPEAN PATENT APPLICATION

(11) **EP 4 620 945 A1**
(43) Date of publication of application: **24.09.2025**
(21) Application number: 23891784.3
(22) Date of filing: 13.09.2023
(51) Int. Cl.: C07C 219/18, C07C 213/02, C07C 213/08

(54) **CATIONIC LIPIDS AND METHOD FOR PREPARING SAME**

(30) Priority: 16.11.2022 KR 20220153897
(71) Applicant: Samyang Holdings Corporation, Seoul 03129 (KR)
(72) Inventor: PARK, Jong Min, Seongnam-si Gyeonggi-do 13590 (KR); LEE, So Jin, Seoul 02468 (KR); SEONG, Shi Hwa, Seoul 06008 (KR); KYUNG, Kyu Jin, Yongin-si Gyeonggi-do 16827 (KR); KIM, Sol, Seongnam-si Gyeonggi-do 13488 (KR); NAM, Joung Pyo, Seoul 02471 (KR); YOON, Yoo Jeong, Seongnam-si Gyeonggi-do 13544 (KR)
(74) Representative: Gleiss Große Schrell und Partner mbB
(86) International application number: PCT/KR2023/013708
(87) International publication number: WO 2024/106715

(57) **Abstract**

The present invention relates to cationic lipids and a method for preparing same, more specifically to cationic lipids that facilitate forming a complex with anionic medicinal material to thus be useful for drug delivery, and to a method for preparing the cationic lipids.

## Description

### TECHNICAL FIELD

The present invention relates to a cationic lipid and a method for preparing the same, and more specifically, the present invention relates to a cationic lipid which can easily form a complex with anionic drug and thus is useful for drug delivery, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. and having many problems in commercialization due to the complexity of the production process. Thus, recent researches proceed in the direction to improve such disadvantages by using non-viral carriers. In comparison with viral carriers, non-viral carriers have advantages of fewer side effects in terms of *in vivo* safety, and lower production cost in terms of economy.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

However, polycation polymers have cytotoxicity due to their multivalent cationic charges, making them problematic for practical use, and conventional cationic lipids used in ionic lipid nanoparticles including cationic lipid, neutral lipid and fusogenic lipid have the disadvantages of complication in the synthesis method, cytotoxicity, and low efficiency of intracellular nucleic acid delivery.

### CONTENTS OF THE INVENTION

### PROBLEMS TO BE SOLVED

The purpose of the present invention is to provide a cationic lipid having a specific structure which can easily form a complex with anionic drug and thus is useful for drug delivery, and a method for preparing the same.

### TECHNICAL MEANS

The first aspect of the present invention provides a lipid having a structure represented by the following formula (1): 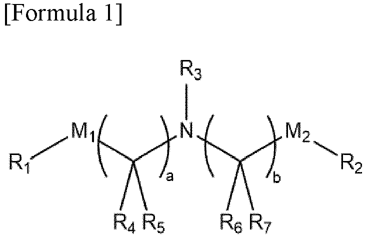 wherein, in the above formula (1),
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

According to an embodiment of the present invention, in the above formula (1), each of M₁ and M₂ may be independently selected from the group consisting of -C(O)O-, -OC(O)-, - OC(O)-M'-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, arylene (more concretely, C₆₋₂₀ arylene), and heteroarylene (more concretely, C₃₋₂₀ heteroarylene having one or more (e.g., 1 to 3) heteroatoms selected from N, O and S), wherein M' may be a direct bond, C₁₋₁₃ alkylene or C₂₋₁₃ alkenylene, and each R' may be independently selected from the group consisting of hydrogen atom, C₁₋₁₈ alkyl and C₂₋₁₈ alkenyl.

According to an embodiment of the present invention, in the above formula (1), each of R₁ and R₂ may be independently selected from the group consisting of C₃₋₂₀ cycloalkyl, C₃₋₂₀ cycloalkenyl, C₆₋₂₀ aryl, C₃₋₂₀ heterocycloalkyl, C₃₋₂₀ heterocycloalkenyl, and C₃₋₂₀ heteroaryl, each of which may be independently unsubstituted or substituted with C₁₋₁₈ alkyl or C₂₋₁₈ alkenyl, wherein each of the heterocycloalkyl, heterocycloalkenyl and heteroaryl may independently have one or more (e.g., 1 to 3) heteroatoms selected from N, O and S.

According to an embodiment of the present invention, in the above formula (1), R₃ may be selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and - CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)2R₁₂, -O(CH2)ₙOR, -N(R)C(=NR₁₃)N(R)₂, -N(R)C(=CHR₁₃)N(R)₂, -OC(O)N(R)₂, - N(R)C(O)OR, -N(OR)C(O)R, -N(OR)S(O)₂R, -N(OR)C(O)OR, -N(OR)C(O)N(R)₂, - N(OR)C(S)N(R)₂, -N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is - (CH₂)_{nQ}, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment of the present invention, in the above formula (1), each of R₄ to R₇ may be independently selected from the group consisting of hydrogen atom, C₁₋₃ alkyl and C₂₋₃ alkenyl.

According to an embodiment of the present invention, in the above formula (1), each of a and b may be independently an integer of from 1 to 15.

More concretely, in the above formula (1), each of M₁ and M₂ may be independently selected from the group consisting of -C(O)O-, -OC(O)-, -OC(O)-M'-C(O)O-, -C(O)N(R')-, - N(R')C(O)- and -C(O)- (wherein M' and R' are the same as defined above).

More concretely, in the above formula (1), each of R₁ and R₂ may be independently selected from the group consisting of C₃₋₂₀ cycloalkyl and C₃₋₂₀ heterocycloalkyl, each of which may be independently unsubstituted or substituted with C₁₋₁₈ alkyl or C₂₋₁₈ alkenyl, wherein the heterocycloalkyl may have one or more (e.g., 1 to 3) heteroatoms selected from N, O and S.

More concretely, in the above formula (1), R₃ may be selected from the group consisting of hydrogen atom, substituted or unsubstituted C₁₋₆ alkyl, and substituted or unsubstituted C₃₋₆ carbocyclic group (e.g., C₃₋₆ cycloalkyl).

More concretely, in the above formula (1), each of R₄ to R₇ may be independently hydrogen atom or C₁₋₃ alkyl.

More concretely, in the above formula (1), each of a and b may be independently an integer of from 3 to 13.

Still more concretely, in the above formula (1), each of M₁ and M₂ may be independently selected from the group consisting of -C(O)O-, -OC(O)-, -C(O)N(R')- and -N(R')C(O)- (wherein R' is the same as defined above).

Still more concretely, in the above formula (1), each of R₁ and R₂ may be independently substituted or unsubstituted C₃₋₁₅ cycloalkyl.

Still more concretely, in the above formula (1), R₃ may be hydrogen atom, or substituted or unsubstituted C₁₋₃ alkyl.

Still more concretely, in the above formula (1), R₄ to R₇ may be hydrogen atom.

Still more concretely, in the above formula (1), each of a and b may be independently an integer of from 5 to 11, and even more concretely, an integer of from 5 to 9.

Even more concretely, the lipid may be one having a structure selected from the following formulas A to R:

| Formula | Structure | Formula | Structure |
|---|---|---|---|
| A | 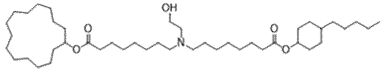 | J | 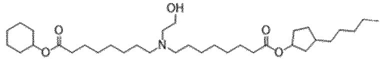 |
| B | 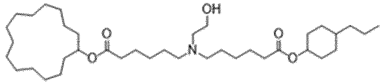 | K | 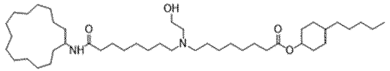 |
| C | 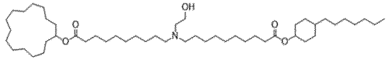 | L | 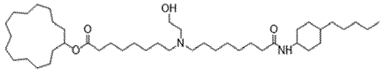 |
| D | 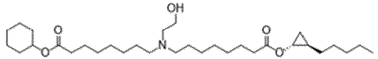 | M | 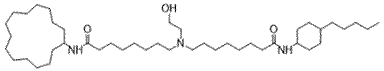 |
| E | 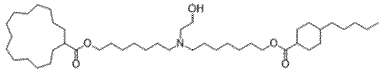 | N | 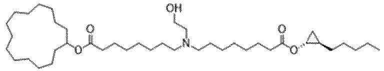 |
| F | 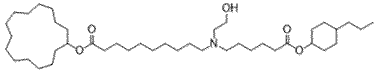 | O | 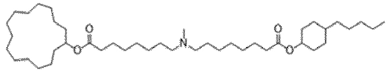 |
| G | 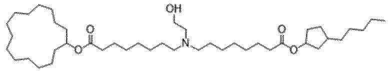 | P | 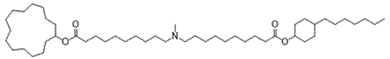 |
| H | 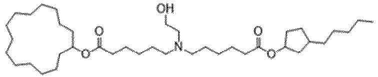 | Q | 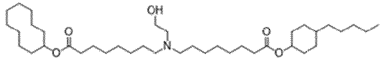 |
| I | 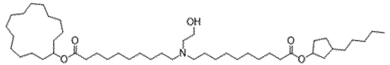 | R | 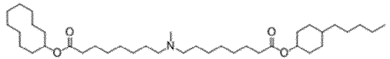 |

The second aspect of the present invention provides a method for preparing a lipid having a structure represented by formula (1'), comprising steps of: (1) reacting a compound of formula (a) with a compound of formula (b) to obtain a compound of formula (c); and (2) reacting a compound of formula (c) with a compound of formula (d):

[Formula a] R₁-OH

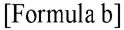

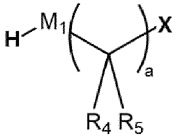

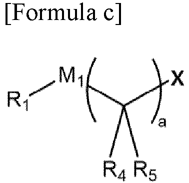

[Formula d] R₃-NH₂

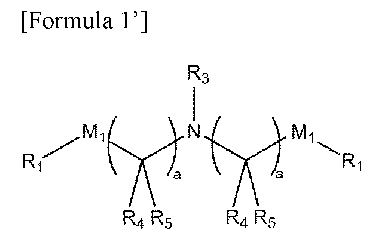

wherein,
M₁, R₁, R₃, R₄, R₅ and a are the same as defined in formula (1) above, and
each X is independently selected from the group consisting of F, CI, Br and I.

The third aspect of the present invention provides a method for preparing a lipid having a structure represented by formula (1), comprising steps of: (1) reacting a compound of formula (a') with a compound of formula (b') to obtain a compound of formula (c'); (2) reacting a compound of formula (c') with a compound of formula (d) to obtain a compound of formula (e); and (3) reacting a compound of formula (e) with a compound of formula (c) obtained from the second aspect of the present invention:

[Formula a'] HO-R₂

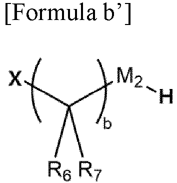

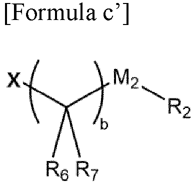

[Formula d] R₃-NH₂

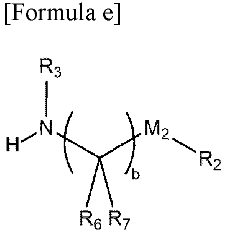

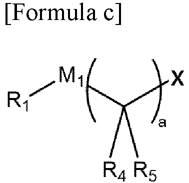

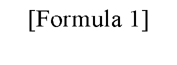

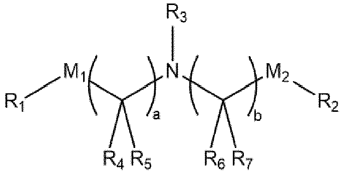

wherein,
M₁, M₂, R₁ to R₇, a and b are the same as defined in formula (1) above, and
each X is independently selected from the group consisting of F, CI, Br and I.

The fourth aspect of the present invention provides a method for preparing a lipid having a structure represented by formula (1'), comprising steps of: (1) reacting a compound of formula (i) with a compound of formula (ii) to obtain a compound of formula (iii); and (2) reacting a compound of formula (iii) with a compound of formula (iv):

[Formula i] R₁-M₁-H

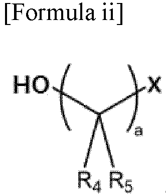

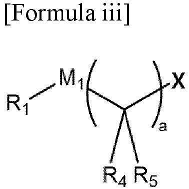

[Formula iv] R₃-NH₂

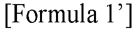

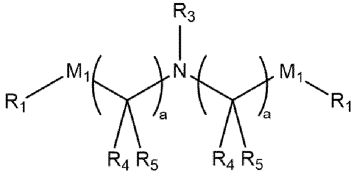

wherein,
M₁, R₁, R₃, R₄, R₅ and a are the same as defined in formula (1) above, and
each X is independently selected from the group consisting of F, CI, Br and I.

The fifth aspect of the present invention provides a method for preparing a lipid having a structure represented by formula (1), comprising steps of: (1) reacting a compound of formula (i') with a compound of formula (ii') to obtain a compound of formula (iii'); (2) reacting a compound of formula (iii') with a compound of formula (iv) to obtain a compound of formula (v); and (3) reacting a compound of formula (v) with a compound of formula (iii) obtained from the fourth aspect of the present invention:

[Formula i'] H-M₂-R₂

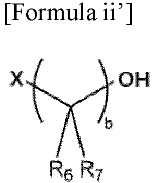

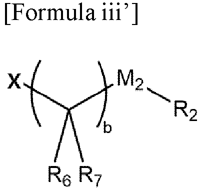

[Formula iv] R₃-NH₂

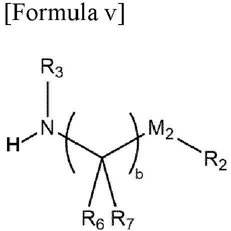

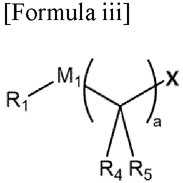

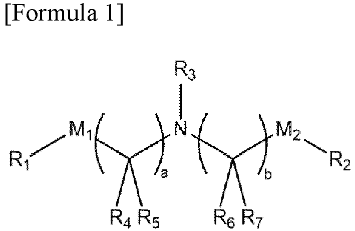

wherein,
M₁, M₂, R₁ to R₇, a and b are the same as defined in formula (1) above, and
each X is independently selected from the group consisting of F, CI, Br and I.

The sixth aspect of the present invention provides a composition for drug delivery comprising the lipid according to the present invention.

### EFFECT OF THE INVENTION

The lipid having a specific structure according to the present invention can easily form a complex with anionic drug, and by utilizing the complex, the drug can be efficiently delivered into the targeted living tissue.

### BRIEF EXPLANATION OF THE DRAWINGS

Figure 1 is a reaction scheme for the lipid synthesis procedure conducted in Example 1.
Figure 2 is a reaction scheme for the lipid synthesis procedure conducted in Example 2.
Figure 3 is a reaction scheme for the lipid synthesis procedure conducted in Example 3.
Figure 4 is a reaction scheme for the lipid synthesis procedure conducted in Example 4.
Figure 5 is a reaction scheme for the lipid synthesis procedure conducted in Example 5.
Figure 6 is a reaction scheme for the lipid synthesis procedure conducted in Example 6.
Figure 7 is a reaction scheme for the lipid synthesis procedure conducted in Example 7.
Figure 8 is a reaction scheme for the lipid synthesis procedure conducted in Example 8.
Figure 9 is a reaction scheme for the lipid synthesis procedure conducted in Example 9.
Figure 10 is a reaction scheme for the lipid synthesis procedure conducted in Example 10.
Figure 11 is a reaction scheme for the lipid synthesis procedure conducted in Example 11.
Figure 12 is a reaction scheme for the lipid synthesis procedure conducted in Example 12.
Figure 13 is a reaction scheme for the lipid synthesis procedure conducted in Example 13.
Figure 14 is a reaction scheme for the lipid synthesis procedure conducted in Example 14.
Figure 15 is a reaction scheme for the lipid synthesis procedure conducted in Example 15.
Figure 16 is a reaction scheme for the lipid synthesis procedure conducted in Example 16.
Figure 17 is a reaction scheme for the lipid synthesis procedure conducted in Example 17.
Figure 18 is a reaction scheme for the lipid synthesis procedure conducted in Example 18.

### CONCRETE MODE FOR CARRYING OUT THE INVENTION

The present invention will be explained in detail below.

A lipid provided by the first aspect of the present invention has a structure represented by the following formula (1): 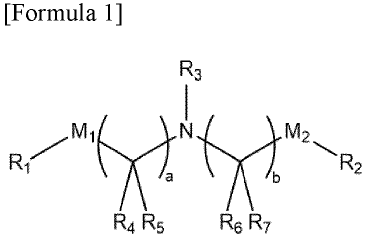 wherein, in the above formula (1),
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

As used herein, the expression "substituted or unsubstituted" for any group means that, unless specified otherwise, the group is not substituted, or is substituted with one or more substituents selected from -OH, halogen atom, C₁₋₈ alkyl group (more concretely, C₃₋₇ alkyl group) or C₁₋₈ halogenated alkyl group (more concretely, C₃₋₇ halogenated alkyl group).

According to an embodiment of the present invention, in the above formula (1), each of M₁ and M₂ may be independently selected from the group consisting of -C(O)O-, -OC(O)-, - OC(O)-M'-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, arylene (more concretely C₆₋₂₀ arylene, still more concretely C₆₋₁₀ arylene), and heteroarylene (more concretely C₃₋₂₀ heteroarylene, still more concretely C₃₋₁₀ heteroarylene, having one or more (e.g., 1 to 3) heteroatoms selected from N, O and S), wherein M' may be a direct bond, C₁₋₁₃ alkylene (more concretely C₁₋₆ alkylene) or C₂₋₁₃ alkenylene (more concretely C₂₋₆ alkenylene), and each R' may be independently selected from the group consisting of hydrogen atom, C₁₋₁₈ alkyl (more concretely C₁₋₁₀ alkyl, still more concretely C₁₋₆ alkyl) and C₂₋₁₈ alkenyl (more concretely C₂₋₁₀ alkenyl, still more concretely C₂₋₆ alkenyl).

According to an embodiment of the present invention, in the above formula (1), each of R₁ and R₂ may be independently selected from the group consisting of substituted or unsubstituted C₃₋₂₀ cycloalkyl (more concretely C₃₋₁₅ cycloalkyl, still more concretely C₆₋₁₅ cycloalkyl), substituted or unsubstituted C₃₋₂₀ cycloalkenyl (more concretely C₃₋₁₅ cycloalkenyl, still more concretely C₆₋₁₅ cycloalkenyl), substituted or unsubstituted C₆₋₂₀ aryl (more concretely C₆₋₁₄ aryl), substituted or unsubstituted C₃₋₂₀ heterocycloalkyl (more concretely C₃₋₁₅ heterocycloalkyl), substituted or unsubstituted C₃₋₂₀ heterocycloalkenyl (more concretely C₃₋₁₅ heterocycloalkenyl), and substituted or unsubstituted C₃₋₂₀ heteroaryl (more concretely C₃₋₁₅ heteroaryl), wherein each of the heterocycloalkyl, heterocycloalkenyl and heteroaryl may independently have one or more (e.g., 1 to 3) heteroatoms selected from N, O and S.

According to an embodiment of the present invention, in the above formula (1), R₃ may be selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and - CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)2R₁₂, -O(CH2)ₙOR, -N(R)C(=NR₁₃)N(R)₂, -N(R)C(=CHR₁₃)N(R)₂, -OC(O)N(R)₂, - N(R)C(O)OR, -N(OR)C(O)R, -N(OR)S(O)₂R, -N(OR)C(O)OR, -N(OR)C(O)N(R)₂, - N(OR)C(S)N(R)₂, -N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is - (CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment of the present invention, in the above formula (1), each of R₄ to R₇ may be independently selected from the group consisting of hydrogen atom, C₁₋₃ alkyl and C₂₋₃ alkenyl.

According to an embodiment of the present invention, in the above formula (1), each of a and b may be independently an integer of from 1 to 15, and even more concretely, an integer of from 3 to 13.

Still more concretely, in the above formula (1), each of M₁ and M₂ may be independently selected from the group consisting of -C(O)O-, -OC(O)-, -C(O)N(R')- and -N(R')C(O)-, wherein R' is the same as defined above.

Still more concretely, in the above formula (1), each of R₁ and R₂ may be independently substituted or unsubstituted C₃₋₁₅ cycloalkyl.

Still more concretely, in the above formula (1), R₃ may be hydrogen atom, or substituted or unsubstituted C₁₋₃ alkyl.

Still more concretely, in the above formula (1), R₄ to R₇ may be hydrogen atom.

Still more concretely, in the above formula (1), each of a and b may be independently an integer of from 5 to 11, and even more concretely, an integer of from 5 to 9.

Even more concretely, the lipid may be one having a structure selected from the following formulas A to R:

| Formula | Structure | Formula | Structure |
|---|---|---|---|
| A | 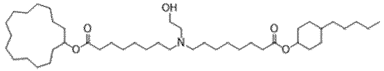 | J | 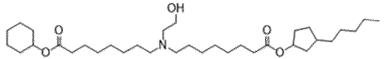 |
| B | 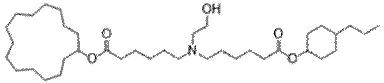 | K | 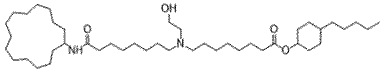 |
| C | 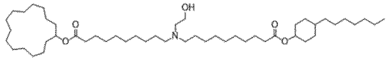 | L | 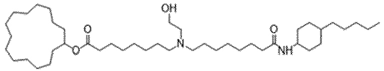 |
| D | 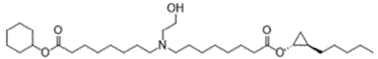 | M | 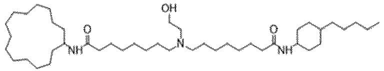 |
| E | 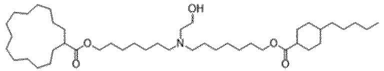 | N | 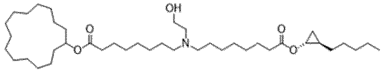 |
| F | 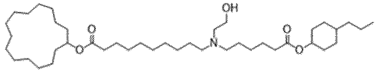 | O | 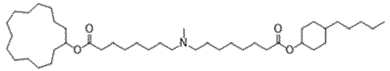 |
| G | 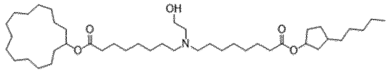 | P | 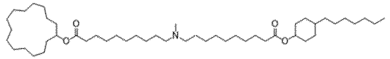 |
| H | 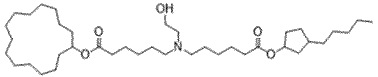 | Q | 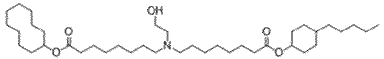 |
| I | 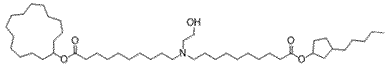 | R | 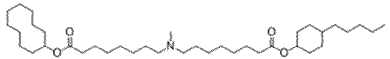 |

The second aspect of the present invention provides a method for preparing a lipid having a structure represented by formula (1') which is included in the above formula (1), comprising steps of: (1) reacting a compound of formula (a) with a compound of formula (b) to obtain a compound of formula (c); and (2) reacting a compound of formula (c) with a compound of formula (d):

[Formula a] R₁-OH

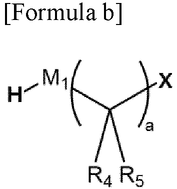

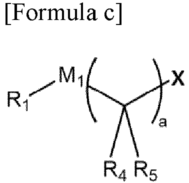

[Formula d] R₃-NH₂

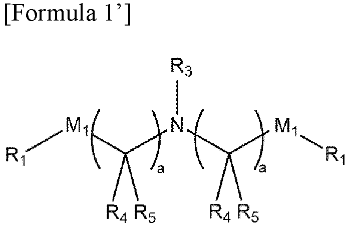

wherein,
M₁, R₁, R₃, R₄, R₅ and a are the same as defined above, and
each X is independently selected from the group consisting of F, CI, Br and I.

In an embodiment of the method for preparing a lipid according to the second aspect of the present invention, the reaction of step (1) may be conducted in a solvent (e.g., methylene chloride (DCM) or toluene) in the presence of a catalyst (e.g., triethylamine (TEA) or sulfuric acid) at room temperature (e.g., from 20 °C to 30 °C) or an elevated temperature (e.g., from 40 °C to 150 °C) condition; and the reaction of step (2) may be conducted in a solvent (e.g., ethanol (EtOH) or dioxane) optionally in the presence of Na₂CO₃ or a catalyst (e.g., N,N-diisopropylethylamine (DIEA)) at an elevated temperature (e.g., from 40 °C to 150 °C) condition, but it is not limited thereto.

According to an embodiment, prior to the reaction of a compound of formula (a) and a compound of formula (b) in step (1), the compound of formula (b) may be pretreated, and this pretreatment may be performed using a chloride compound (e.g., oxalyl chloride) in the presence of dimethylformamide (DMF) in a solvent (e.g., methylene chloride (DCM)) at a low temperature (e.g., -10 °C to 10 °C) or room temperature (e.g., 20 °C to 30 °C) condition, but it is not limited thereto.

The third aspect of the present invention provides a method for preparing a lipid having a structure represented by the above formula (1), comprising steps of: (1) reacting a compound of formula (a') with a compound of formula (b') to obtain a compound of formula (c'); (2) reacting a compound of formula (c') with a compound of formula (d) to obtain a compound of formula (e); and (3) reacting a compound of formula (e) with a compound of formula (c) obtained from the second aspect of the present invention:

[Formula a'] HO-R₂

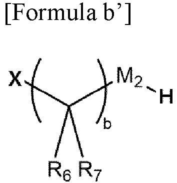

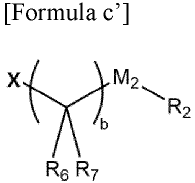

[Formula d] R₃-NH₂

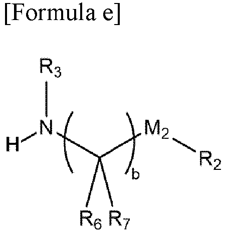

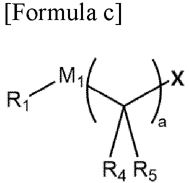

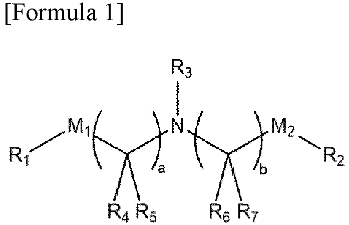

wherein,
M₁, M₂, R₁ to R₇, a and b are the same as defined above, and
each X is independently selected from the group consisting of F, CI, Br and I.

In an embodiment of the method for preparing a lipid according to the third aspect of the present invention, the reaction of step (1) may be conducted in a solvent (e.g., methylene chloride (DCM) or toluene) in the presence of a catalyst (e.g., triethylamine (TEA) or sulfuric acid) at room temperature (e.g., from 20 °C to 30 °C) or an elevated temperature (e.g., from 40 °C to 150 °C) condition; and each of the reactions of steps (2) and (3) may be independently conducted in a solvent (e.g., ethanol (EtOH)) optionally in the presence of Na₂CO₃ or a catalyst (e.g., N,N-diisopropylethylamine (DIEA)) at an elevated temperature (e.g., from 40 °C to 150 °C) condition, but it is not limited thereto.

According to an embodiment, prior to the reaction of a compound of formula (a') and a compound of formula (b') in step (1), the compound of formula (b') may be pretreated, and this pretreatment may be performed using a chloride compound (e.g., oxalyl chloride) in the presence of dimethylformamide (DMF) in a solvent (e.g., methylene chloride (DCM)) at a low temperature (e.g., -10 °C to 10 °C) or room temperature (e.g., 20 °C to 30 °C) condition, but it is not limited thereto.

The fourth aspect of the present invention provides a method for preparing a lipid having a structure represented by the above formula (1'), comprising steps of: (1) reacting a compound of formula (i) with a compound of formula (ii) to obtain a compound of formula (iii); and (2) reacting a compound of formula (iii) with a compound of formula (iv):

[Formula i] R₁-M₁-H

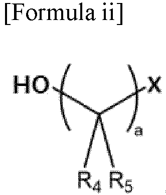

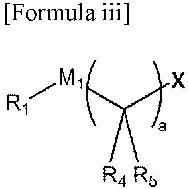

[Formula iv] R₃-NH₂

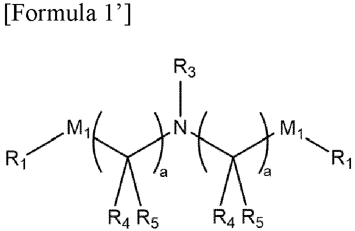

wherein,
M₁, R₁, R₃, R₄, R₅ and a are the same as defined above, and
each X is independently selected from the group consisting of F, CI, Br and I.

In an embodiment of the method for preparing a lipid according to the fourth aspect of the present invention, the reaction of step (1) may be conducted in a solvent (e.g., methylene chloride (DCM) or toluene) in the presence of a catalyst (e.g., triethylamine (TEA) or sulfuric acid) at room temperature (e.g., from 20 °C to 30 °C) or an elevated temperature (e.g., from 40 °C to 150 °C) condition; and the reaction of step (2) may be conducted in a solvent (e.g., ethanol (EtOH) or dioxane) optionally in the presence of Na₂CO₃ or a catalyst (e.g., N,N-diisopropylethylamine (DIEA)) at an elevated temperature (e.g., from 40 °C to 150 °C) condition, but it is not limited thereto.

The fifth aspect of the present invention provides a method for preparing a lipid having a structure represented by the above formula (1), comprising steps of: (1) reacting a compound of formula (i') with a compound of formula (ii') to obtain a compound of formula (iii'); (2) reacting a compound of formula (iii') with a compound of formula (iv) to obtain a compound of formula (v); (3) reacting a compound of formula (v) with a compound of formula (iii) obtained from the fourth aspect of the present invention:

[Formula i'] H-M₂-R₂

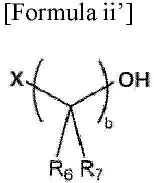

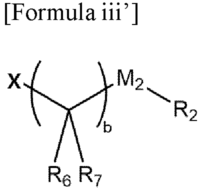

[Formula iv] R₃-NH₂

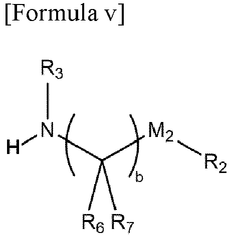

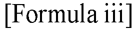

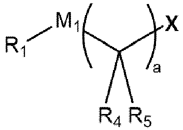

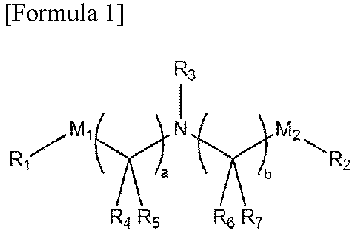

wherein,
M₁, M₂, R₁ to R₇, a and b are the same as defined above, and
each X is independently selected from the group consisting of F, CI, Br and I.

In an embodiment of the method for preparing a lipid according to the fifth aspect of the present invention, the reaction of step (1) may be conducted in a solvent (e.g., methylene chloride (DCM) or toluene) in the presence of a catalyst (e.g., triethylamine (TEA) or sulfuric acid) at room temperature (e.g., from 20 °C to 30 °C) or an elevated temperature (e.g., from 40 °C to 150 °C) condition; and each of the reactions of steps (2) and (3) may be independently conducted in a solvent (e.g., ethanol (EtOH)) optionally in the presence of Na₂CO₃ or a catalyst (e.g., N,N-diisopropylethylamine (DIEA)) at an elevated temperature (e.g., from 40 °C to 150 °C) condition, but it is not limited thereto.

A lipid having a structure represented by formula 1 of the present invention can easily form a complex with anionic drug and thus is useful for drug delivery.

Thus, the sixth aspect of the present invention provides a composition for drug delivery comprising a lipid having a structure represented by formula 1 of the present invention.

In an embodiment, the drug may be selected from nucleic acid, polypeptide, virus or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

In an embodiment, the lipid of the present invention may form a complex with the drug, and the complex is encapsulated within nanoparticle structure formed by an amphiphilic block copolymer.

In an embodiment, the amphiphilic block copolymer may be an A-B type block copolymer comprising a hydrophilic A block and a hydrophobic B block. In an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More concretely, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More concretely, the hydrophobic B block may be one or more selected from the group consisting of polylactide (PLA), polyglycolide, polycaprolactone, polydioxan-2-one, copolymer of polylactide and glycolide, copolymer of polylactide and polydioxan-2-one, copolymer of polylactide and and polycaprolactone, and a copolymer of polyglycolide and polycaprolactone.

Also, in an embodiment, in order to increase the hydrophobicity of the hydrophobic B block and thereby improve the stability of the nanoparticle, the hydrophobic B block may be modified by chemically combining the hydroxyl group at the end of the hydrophobic B block with tocopherol, cholesterol, or fatty acid having 10 to 24 carbons.

The present invention will be explained below in more detail with reference to the following Examples. However, the Examples are only to illustrate the invention, and the scope of the present invention is not limited thereby in any manner.

### EXAMPLES

### Example 1

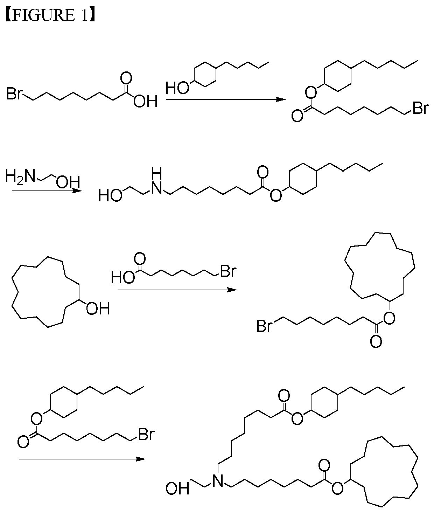

### 1-1. The compound of the following formula A was prepared according to the synthesis scheme shown in Figure 1.

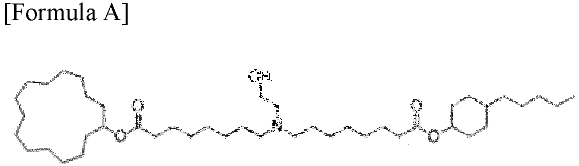

### 1-2. Synthesis of 4-pentylcyclohexyl 8-bromooctanoate

In a 250 mL 3-neck round bottom flask (RBF), 8-bromooctanoic acid (2.00 g, 8.96 mmol, 1.00 eq), dichloromethane (DCM) (40 mL), and dimethylformamide (DMF) (0.5 mL) were added together, and oxalyl chloride (2.28 g, 17.9 mmol, 2.00 eq) was added thereto at 0 °C under a nitrogen environment. The resulting mixture was stirred at 25 °C for 4 hours under a nitrogen environment, and then 4-pentylcyclohexan-1-ol (2.29 g, 13.5 mmol, 1.50 eq) and triethylamine (TEA) (1.36 g, 13.5 mmol, 1.50 eq) were added, and the mixture was stirred at 25 °C for an additional 12 hours under a nitrogen environment. The reaction mixture was concentrated in vacuo, and the concentrated residue was purified using a silica column with petroleum ether:ethyl acetate (EtOAc) = 1:0 → 50:1 to obtain 4-pentylcyclohexyl 8-bromooctanoate (2.46 g, 6.55 mmol, 73.1% yield) as a pale yellow oil.
¹H NMR (400 MHz, CHLOROFORM-d): δ 0.76 - 0.86 (m, 3 H) 0.88 - 1.94 (m, 28 H) 2.21 (dt, 2 H) 3.33 (td, 2 H) 4.52 - 4.66 (m, 1 H) 4.87 - 4.95 (m, 1 H)

### 1-3. Synthesis of 4-pentylcyclohexyl 8-((2-hydroxyethyl)amino)octanoate

In a 100 mL 3-neck RBF, 4-pentylcyclohexyl 8-bromooctanoate (2.46 g, 6.55 mmol, 1.00 eq), 2-aminoethan-1-ol (2.00 g, 32.8 mmol, 5.00 eq), and ethanol (EtOH) (50 mL) were added, and the mixture was stirred at 80 °C for 16 hours under a nitrogen environment. The reaction mixture was concentrated in vacuo, and the concentrated residue was purified using a silica column with petroleum ether:EtOAc = 1:0 → 50:1 to obtain 4-pentylcyclohexyl 8-((2-hydroxyethyl)amino)octanoate (2.00 g, 5.62 mmol, 85.8 % yield) as a yellow solid.
¹H NMR (400 MHz, CHLOROFORM-d): δ 0.76 - 0.86 (m, 3 H) 0.88 - 1.94 (m, 27 H) 2.32 (t, 2 H) 2.51 (t, 2 H) 2.71 (t, 2H), 3.54 (d, 2 H) 3.91 - 4.00 (m, 1 H) 4.11 - 4.31 (m, 1 H)

### 1-4. Synthesis of cyclopentadecyl 8-bromooctanoate

In a 250 mL 3-neck RBF, cyclopentadecanol (5.00 g, 22.1 mmol, 1.00 eq), 8-bromooctanoic acid (4.93 g, 22.1 mmol, 1 eq), sulfuric acid (H2SO4) (217 mg, 2.21 mmol, 0.10 eq), and toluene (100 mL) were added, and the mixture was stirred at 120 °C for 16 hours under a nitrogen environment. After evaporating the solvent, the residue was purified using a silica column with petroleum ether:EtOAc = 1:0 → 50:1 to obtain cyclopentadecyl 8-bromooctanoate (2.60 g, 6.03 mmol, 27.3% yield) as a colorless oil.
¹H NMR (400 MHz, CHLOROFORM-d): δ 4.89 (quin, 1H), 3.41 (dt, 2H), 2.28 (t, 2H), 1.85 (quin, 2H), 1.73 - 1.16 (m, 36H)

### 1-5. Synthesis of the compound of formula A

In a 100 mL 3-neck RBF, cyclopentadecyl 8-bromooctanoate (1.60 g, 3.71 mmol, 1.00 eq), 4-pentylcyclohexyl 8-((2-hydroxyethyl)amino)octanoate (1.32 g, 3.71 mmol, 1.00 eq), N,N-diisopropylethylamine (DIEA) (527 mg, 4.08 mmol, 1.10 eq), and EtOH (30 mL) were added, and the mixture was stirred at 80 °C for 48 hours. Subsequently, after evaporating the solvent, the residue was purified using a silica column with petroleum ether:EtOAc = 10:1 → 1:1, and purified again by prep-HPLC (Folic Acid condition) and washed with NaHCO₃ aqueous solution (300 mL), and then the organic layer was concentrated and extracted with DCM (200 mL x 2). The organic layer was dried over anhydrous Na₂SO₄ and filtered, and the filtrate was concentrated to obtain the compound of formula A (0.240 g, 340 µmol, 9.16% yield) as a yellow oil.
¹H NMR (400 MHz, CHLOROFORM-d): δ 4.91 (br s, 1H), 4.82 (quin, 1H), 3.46 (br t, 2H), 2.51 (br d, 2H), 2.37 (br t, 4H), 2.21 (td, 4H), 1.58 - 1.42 (m, 14H), 1.33 - 1.17 (m, 52H), 0.83 - 0.80 (m, 3H).

### Example 2

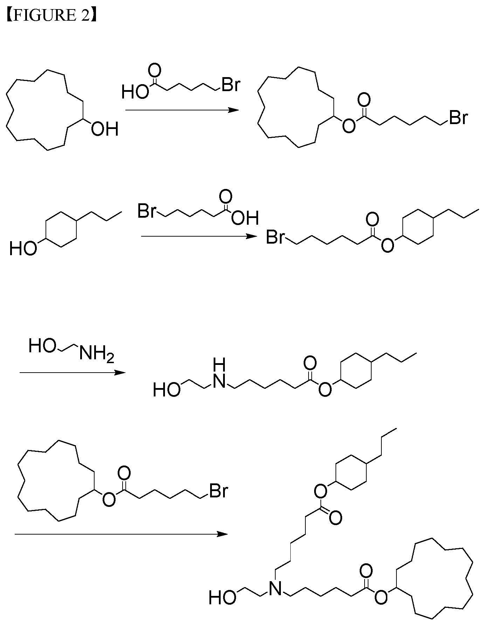

### 2-1. The compound of the following formula B was prepared according to the synthesis scheme shown in Figure 2.

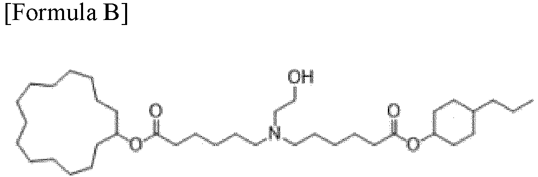

### 2-2. Synthesis of cyclopentadecyl 6-bromohexanoate

In a 100 mL 3-neck RBF, cyclopentadecanol (5.00 g, 22.1 mmol, 1.00 eq), 6-bromohexanoic acid (6.46 g, 33.1 mmol, 1.50 eq), 1-ethyl-3-(3-dimethylaminopropyl)carbodiimide (EDCI) (5.08 g, 26.5 mmol, 1.20 eq), 4-dimethylaminopyridine (DMAP) (540 mg, 4.42 mmol, 0.20 eq), TEA (4.47 g, 44.2 mmol, 6.15 mL, 2.00 eq) and DCM (50 mL) were added, and the mixture was purged three times with nitrogen gas. The resulting mixture was stirred at 25 °C for 16 hours under a nitrogen environment, and after terminating the reaction by adding 200 mL of water at 20 °C, the mixture was extracted with 600 mL of DCM (200 mL x 3). The organic layers were collected, dried over Na₂SO₄, filtered, and the filtrate was concentrated in vacuo. The concentrated residue was purified using a silica column with petroleum ether:EtOAc = 100:1 → 1:1 to give cyclopentadecyl 6-bromohexanoate (2.20 g, 5.45 mmol, 24.7% yield) as a yellow oil.
¹H NMR (400 MHz, CHLOROFORM-d): δ 4.83 (quin, 1H), 3.50 - 3.30 (m, 2H), 2.23 (t, 2H), 1.87 - 1.68 (m, 2H), 1.63 - 1.37 (m, 10H), 1.26 (br s, 22H)

### 2-3. Synthesis of 4-propylcyclohexyl 6-bromohexanoate

In a 250 mL 3-neck RBF, 4-propylcyclohexan-1-ol (10.0 g, 70.3 mmol, 1.00 eq), 6-bromohexanoic acid (16.5 g, 84.4 mmol, 1.20 eq), EDCI (20.2 g, 105 mmol, 1.50 eq), DMAP (8.59 g, 70.3 mmol, 1.00 eq), and TEA (7.11 g, 70.3 mmol, 9.79 mL, 1.00 eq) in DCM (100 mL) were added, and the mixture was purged three times with nitrogen, and stirred at 25 °C for 16 hours under a nitrogen environment. The reaction mixture was neutralized by adding 100 mL of water at 20 °C and extracted with 600 mL of DCM (200 mL x 3), and then the organic layers were collected and concentrated in vacuo, and the concentrated residue was purified using a silica column with petroleum ether:EtOAc = 100:1 → 10:1 to give 4-propylcyclohexyl 6-bromohexanoate (6.80 g, 21.3 mmol, 30.3% yield) as a yellow oil.
¹H NMR (400 MHz, CHLOROFORM-d): δ 0.76 - 0.86 (m, 3 H) 0.88 - 1.94 (m, 19 H) 2.21 (dt, 2 H) 3.33 (td, 2 H) 4.52 - 4.66 (m, 1 H)

### 2-4. Synthesis of 4-propylcyclohexyl 6-((2-hydroxyethyl)amino)hexanoate

In a 250 mL 3-neck RBF, 4-propylcyclohexyl 6-bromohexanoate (3.00 g, 9.40 mmol, 1.00 eq), 2-aminoethanol-1-ol (2.87 g, 47.0 mmol, 2.84 mL, 5.00 eq), and EtOH (60 mL) were added, and the mixture was purged three times with nitrogen, and stirred at 85 °C for 16 hours under a nitrogen environment. The reaction mixture was cooled to room temperature and concentrated to remove the solvent, and the residue was purified by column chromatography (SiO₂, DCM:MeOH = 100:1 → 1:1) to give 4-propylcyclohexyl 6-((2-hydroxyethyl)amino)hexanoate (0.70 g, 2.34 mmol, 24.9% yield) as a yellow oil.
¹H NMR (400 MHz, CHLOROFORM-d): δ 5.04 - 4.60 (m, 1H), 3.70 - 3.61 (m, 2H), 2.79 (t, 2H), 2.64 (dt, 2H), 2.35 - 2.24 (m, 2H), 2.01 - 1.94 (m, 2H), 1.87 - 1.74 (m, 2H), 1.65 (qd, 2H), 1.56 - 1.48 (m, 4H), 1.41 - 1.16 (m, 9H), 1.07 - 0.94 (m, 1H), 0.89 (dt, 3H)

### 2-5. Synthesis of the compound of formula B

In a 50 mL 3-neck RBF, 4-propylcyclohexyl 6-((2-hydroxyethyl)amino)hexanoate (371 mg, 1.24 mmol, 1.00 eq) and 4-propylcyclohexyl 6-bromohexanoate (0.50 g, 1.24 mmol, 1.00 eq), N,N-diisopropylethylamine (DIEA) (800 mg, 6.20 mmol, 1.08 mL, 5 eq) were added together with 1,4-dioxane (10 mL), and the mixture was purged three times with nitrogen, and stirred at 95 °C for 16 hours in a nitrogen environment. The reaction mixture was cooled to room temperature and concentrated under reduced pressure to remove the solvent, and the residue was purified through column chromatography (SiO₂, DCM:MeOH = 100:1 → 10:1) to obtain the compound of formula B (0.21 g, 337.64 µmol, 27.2% yield) as a yellow oil.
¹H NMR (400 MHz, CHLOROFORM-d): δ 4.94 - 4.52 (m, 2H), 3.79 (br s, 2H), 3.06 - 2.63 (m, 6H), 2.29 - 2.17 (m, 4H), 1.93 - 1.81 (m, 1H), 1.79 - 1.37 (m, 17H), 1.36 - 1.09 (m, 34H), 0.99 - 0.86 (m, 1H), 0.85 - 0.76 (m, 3H)

### Example 3

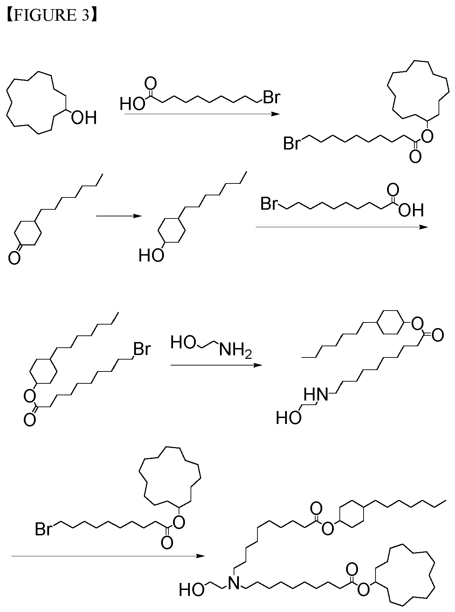

### 3-1. The compound of the following formula C was prepared according to the synthesis scheme shown in Figure 3.

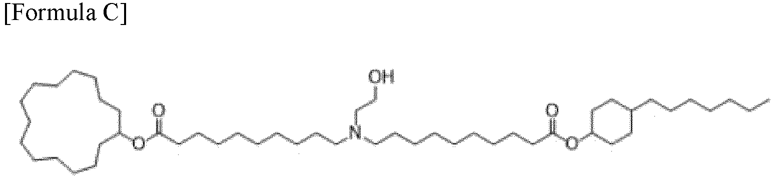

### 3-2. Synthesis of cyclopentadecyl 10-bromodecanoate

In a 100 mL 3-neck RBF, cyclopentadecanol (5.00 g, 22.1 mmol, 1.00 eq), 10-bromodecanoic acid (8.32 g, 33.1 mmol, 1.50 eq), DMAP (540 mg, 4.42 mmol, 0.20 eq), EDCI (5.08 g, 26.5 mmol, 1.20 eq) and TEA (4.47 g, 44.2 mmol, 2.00 eq) were added together with DCM (50 mL), and the mixture was purged three times with nitrogen, and stirred at 50 °C for 16 hours in a nitrogen environment. The reaction mixture was cooled to 20 °C and water was added to terminate the reaction, and extracted with 600 mL of DCM (200 mL x 3), and then the organic layers were collected, dried over Na₂SO₄, filtered, and the filtrate was concentrated. The residue obtained was purified by column chromatography (SiO₂, petroleum ether:EtOAc = 100:1 → 1:1) to give cyclopentadecyl 10-bromodecanoate (2.60 g, 5.66 mmol, 25.6% yield) as a yellow oil.
¹H NMR (400 MHz, CHLOROFORM-d): δ 4.90 (quin, 1H), 3.58 - 3.36 (m, 2H), 2.27 (t, 2H), 1.90 - 1.72 (m, 2H), 1.64 - 1.53 (m, 6H), 1.43 - 1.28 (m, 34H)

### 3-3. Synthesis of 4-heptylcyclohexan-1-ol

In a 500 mL 3-neck RBF, LiAlH₄ (2.50 M in THF, 61.1 mL, 1.20 eq) was added together with THF (250 mL), the mixture was cooled to 0 °C, and a solution of 4-heptylcyclohexan-1-one (25.0 g, 127 mmol, 1.00 eq) in THF (250 mL) was slowly added thereto over 20 minutes in a nitrogen environment. The temperature of the mixture solution was then increased to 25 °C, and the mixture was stirred for 3 hours in a nitrogen environment. The reaction mixture was cooled to 0 °C and then, in a nitrogen environment, water (120 mL) was added, paying attention not to generate a large amount of foam. Then, 15% aq. NaOH (12 mL) was slowly added to the reaction mixture at 0 °C, and after 5 min, water (36 mL) was added at the same temperature, and the temperature was slowly raised to 25 °C and the mixture was stirred for 15 min. The resulting mixture was filtered, and the filtered cake was concentrated in vacuo to give 4-heptylcyclohexan-1-ol (50.0 g, 252 mmol, 98.9% yield) as a colorless powder.
¹H NMR (400 MHz, CHLOROFORM-*d*): *δ* 3.47 (tt, H), 1.95 - 1.84 (m, 2H), 1.75 - 1.65 (m, 2H), 1.42 (s, 1H), 1.24 - 1.14 (m, 14H), 1.11 - 1.08 (m, 2H), 0.83 - 0.80 (m, 3H)

### 3-4. Synthesis of 4-heptylcyclohexyl 10-bromodecanoate

In a 250 mL 3-neck RBF, 4-heptylcyclohexan-1-ol (10.0 g, 50.4 mmol, 1.00 eq), 10-bromodecanoic acid (15.2 g, 60.5 mmol, 1.20 eq), EDCI (14.5 g, 75.6 mmol, 1.50 eq), DMAP (6.16 g, 50.4 mmol, 1.00 eq), TEA (5.10 g, 50.4 mmol, 1.00 eq) and DCM (100 mL) were added, and the mixture was purged three times with nitrogen, and stirred at 25 °C for 16 hours in a nitrogen environment. The reaction mixture was neutralized by adding 200 mL of water at 20 °C and extracted with 600 mL of DCM (200 mL x 3), and then the organic layers were collected and concentrated in vacuo, and the concentrated residue was purified by column chromatography (SiO₂, petroleum ether:EtOAc = 100:1 → 1:1) to give 4-heptylcyclohexyl 10-bromodecanoate (6.60 g, 15.3 mmol, 30.3% yield) as a white solid.
¹H NMR (400 MHz, CHLOROFORM-d): *δ* 4.59 (tt, 1H), 3.46 (t, 1H), 3.33 (t, 1H), 2.19 (t, 2H), 1.87 (br dd, 2H), 1.82 - 1.67 (m, 4H), 1.58 - 1.50 (m, 2H), 1.38 - 1.32 (m, 2H), 1.21 (br d, 23H), 0.97 - 0.86 (m, 2H), 0.81 (t, 3H)

### 3-5. Synthesis of 4-heptylcyclohexyl 10-((2-hydroxyethyl)amino)decanoate

In a 250 mL 3-neck RBF, 4-heptylcyclohexyl 10-bromodecanoate (2.30 g, 5.33 mmol, 1.00 eq) and 2-aminoethanol-1-ol (1.63 g, 26.6 mmol, 5 eq) were added together with EtOH (60 mL), and the mixture was purged three times with nitrogen, and stirred at 95 °C for 16 hours under a nitrogen environment. The reaction mixture was concentrated in vacuo, and the concentrated residue was purified by column chromatography (SiO₂, DCM:MeOH = 100:1 → 1:1) to give 4-heptylcyclohexyl 10-((2-hydroxyethyl)amino)decanoate (1.30 g, 3.16 mmol, 59.2% yield) as a white solid.
¹H NMR (400 MHz, CHLOROFORM-d): *δ* 4.66 (tt, 1H), 3.83 - 3.73 (m, 2H), 2.96 - 2.88 (m, 2H), 2.81 - 2.73 (m, 2H), 2.26 (t, 2H), 1.95 (br dd, 2H), 1.78 (br d, 2H), 1.62 (td, 4H), 1.28 (br d, 24H), 1.18 (br d, 2H), 1.04 - 0.96 (m, 2H), 0.89 (t, 3H)

### 3-6. Synthesis of the compound of formula C

In a 50 mL 3-neck RBF, 4-heptylcyclohexyl 10-((2-hydroxyethyl)amino)decanoate (0.80 g, 1.94 mmol, 1.00 eq), cyclopentadecyl 10-bromodecanoate (893 mg, 1.94 mmol, 1.00 eq), and DIEA (1.26 g, 9.72 mmol, 1.69 mL, 5.00 eq) were added together with EtOH (3 mL). The mixture was purged three times with nitrogen and stirred at 95 °C for 16 hours under a nitrogen environment. The reaction mixture was cooled to room temperature, concentrated in vacuo, and the concentrated residue was purified by chromatography (SiO₂, DCM:MeOH = 100:1 → 10:1) to obtain the compound of formula C (0.35 g, 447 µmol, 22.9% yield) as a white solid.
¹H NMR (400 MHz, CHLOROFORM-*d*): *δ* 4.96 - 4.61 (m, 2H), 3.77 - 3.52 (m, 2H), 2.84 - 2.37 (m, 6H), 2.27 (dt, 4H), 2.01 - 1.92 (m, 2H), 1.79 (br d, 2H), 1.68 - 1.46 (m, 18H), 1.37 - 1.25 (m, 56H), 1.00 (br d, 1H), 0.89 (br t, 3H)

### Example 4

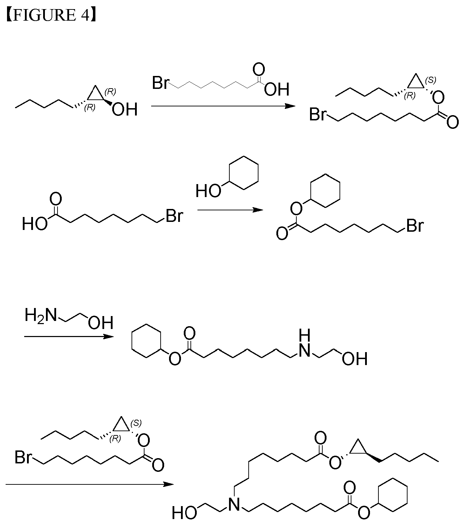

### 4-1. The compound of the following formula D was prepared according to the synthesis scheme shown in Figure 4.

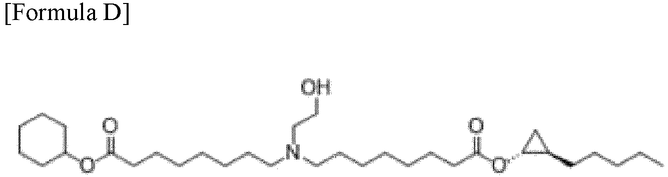

### 4-2. Synthesis of (E)-2-(hept-1-en-1-yl)-4,4,5,5-tetramethyl-1,3,2-dioxaborolane

In a 500 mL 3-neck RBF, hept-1-yne (64.4 g, 670 mmol, 1.00 eq), TEA (Et3N, 6.78 g, 670 mmol, 0.10 eq), 4,4,5,5-tetramethyl-1,3,2-dioxaborolane (90.0 g, 703 mmol, 1.05 eq), chlorozirconium and cyclopentane (17.9 g, 670 mmol, 0.10 eq) were added, and the mixture was purged three times with nitrogen and then stirred at 60 °C for 16 hours in a nitrogen environment. The reaction mixture was cooled to room temperature and filtered, and the filtrate was concentrated in vacuo, and the concentrated residue was purified by silica column chromatography (petroleum ether:EtOAc = 10:1) to give (E)-2-(hept-1-en-1-yl)-4,4,5,5-tetramethyl-1,3,2-dioxaborolane (102 g, 455 mmol, 67.9% yield) as a yellow oil.
¹H NMR: (400 MHz, CHLOROFORM-*d*): δ 6.64 (td, 1H), 5.43 (td, 1H), 2.19 - 2.12 (m, 2H), 1.44 - 1.39 (m, 2H), 1.30 (br d, 4H), 1.27 (s, 12H), 0.90 - 0.87 (m, 3H)

### 4-3. Synthesis of 4,4,5,5-tetramethyl-2-((1R,2R)-2-pentylcyclopropyl)-1,3,2-dioxaborolane

In a 1000 mL 3-neck RBF, diethyl zinc (1 M, 178.5 mL, 2.00 eq) was added together with distilled DCM (80 mL) purified under a nitrogen environment, and a solution of trifluoroacetic acid (TFA) (20.4 g, 178 mmol, 2.00 eq) dissolved in DCM (40 mL) was added little by little at 0 °C. In addition, a solution of diiodomethane (47.8 g, 178 mmol, 2.00 eq) dissolved in DCM (40 mL) was added with stirring at 0 °C for 30 min. The reaction mixture was stirred for an additional 30 min, then a solution of (E)-2-(hept-1-en-1-yl)-4,4,5,5-tetramethyl-1,3,2-dioxaborolane (20.0 g, 89.2 mmol, 1.00 eq) in DCM (40 mL) was added at 0 °C, and the mixture was stirred at 25 °C for 2 hours in a nitrogen environment. The reaction mixture was neutralized with water and extracted with DCM (1000 mL x 3), and the organic layers were collected, dried over Na₂SO₄ and filtered, and the filtrate was concentrated in vacuo and the concentrated residue was purified by silica column chromatography (petroleum ether:EtOAc = 10:1 → 1: 100) to give 4,4,5,5-tetramethyl-2-((1R,2R)-2-pentylcyclopropyl)-1,3,2-dioxaborolane (14.0 g, 58.8 mmol, 65.9% yield) as a yellow oil.
¹H NMR (400 MHz, CHLOROFORM-*d*): δ 1.42 - 1.37 (m, 2H), 1.32 - 1.26 (m, 6H), 1.26 - 1.24 (m, 2H), 1.22 (s, 10H), 0.89 (br t, 4H), 0.67 (dt, 1H), 0.43 - 0.33 (m, 1H), -0.42 (td, 1H)

### 4-4. Synthesis of (1R,2R)-2-pentylcyclopropan-1-ol

In a 500 mL 3-neck RBF, 4,4,5,5-tetramethyl-2-((1R,2R)-2-pentylcyclopropyl)-1,3,2-dioxaborolane (8.00 g, 33.6 mmol, 1.00 eq) and THF (160 mL) were added, and to the mixture, NaOH (2.69 g, 67.2 mmol, 2.00 eq) was slowly added at 0 °C and H₂O₂ (7.87 g, 69.4 mmol, 30% purity, 2.07 eq) was added at 0 °C, and then the mixture was stirred at 25 °C for 16 hours. Then, Na₂SO₃ (2 eq) ice-water was poured into the reaction mixture to terminate the reaction, followed by extraction with DCM (30 mL x 3), and the organic layers were collected, dried over Na₂SO₄ and filtered, the filtrate was concentrated in vacuo, and the concentrated residue was purified by silica column chromatography (petroleum ether:EtOAc = 10:1 → 1:100) to obtain (1R,2R)-2-pentylcyclopropan-1-ol (2.50 g, 19.5 mmol, 58.1% yield) as a yellow oil.
¹H NMR (400 MHz, CHLOROFORM-*d*): *δ* 3.20 (td, 1H), 1.84 - 1.67 (m, 1H), 1.41 - 1.35 (m, 2H), 1.33 - 1.26 (m, 4H), 1.23 - 1.06 (m, 2H), 0.94 - 0.87 (m, 4H), 0.68 (ddd, 1H), 0.31 (q, 1H)

### 4-5. Synthesis of (1R,2R)-2-pentylcyclopropyl 8-bromooctanoate

In a 100 mL 3-neck RBF, (1R,2R)-2-pentylcyclopropan-1-ol (2.50 g, 19.5 mmol, 1.00 eq) was added together with DCM (25 mL), and 8-bromooctanoic acid (5.22 g, 23.4 mmol, 1.20 eq), EDCI (4.49 g, 23.4 mmol, 1.20 eq), DMAP (476 mg, 3.90 mmol, 0.20 eq) and Et3N (3.95 g, 39.00 mmol, 2.00 eq) were added. The mixture was purged three times with nitrogen, and stirred at 25 °C for 16 hours in a nitrogen environment. The reaction mixture was filtered and the filtrate was concentrated in vacuo, and the concentrated residue was purified by silica column chromatography (petroleum ether:EtOAc = 10:1 → 1:100) to give (1R,2R)-2-pentylcyclopropyl 8-bromooctanoate (3.50 g, 10.5 mmol, 53.9% yield) as a yellow oil.
¹H NMR (400 MHz, CHLOROFORM-*d*): δ 3.85 - 3.79 (m, 1H), 3.41 (t, 2H), 2.26 (t, 2H), 1.86 (quin, 2H), 1.65 - 1.58 (m, 2H), 1.46 - 1.38 (m, 4H), 1.36 - 1.27 (m, 10H), 1.00 (ddd, 1H), 0.89 (br t, 3H), 0.78 (ddd, 1H), 0.52 (q, 1H)

### 4-6. Synthesis of cyclohexyl 8-bromooctanoate

In a 1000 mL 3-neck RBF, 8-bromooctanoic acid (15.0 g, 67.2 mmol, 1.00 eq) was placed together with DCM (150 mL), and trifluoroacetic anhydride (TFAA) (14.1 g, 67.2 mmol, 1.00 eq) was added in a nitrogen environment, and the mixture was stirred at 0-25 °C for 2.5 hours in a nitrogen environment. To the mixture, cyclohexanol (33.7 g, 336 mmol, 35.1 mL, 5.00 eq) was added at 0 °C, and the mixture was stirred at 25 °C for 16 hours in a nitrogen environment, and the reaction mixture was neutralized with 100 mL of water at 20 °C, and extracted with 600 mL of DCM (200 mL x 3). The organic layers were collected, dried over Na₂SO₄ and filtered, and the filtrate was concentrated in vacuo, and the concentrated residue was purified by silica column chromatography (SiO₂, petroleum ether:EtOAc = 100:1 → 10:1) to give cyclohexyl 8-bromooctanoate (7.10 g, 23.3 mmol, 34.6% yield) as a colorless oil.
¹H NMR (400 MHz, CHLOROFORM-*d*): *δ* 4.77 (br d, 1H), 3.42 (t, 2H), 2.30 (t, 2H), 1.93 - 1.82 (m, 4H), 1.74 (br dd, 2H), 1.67 - 1.55 (m, 4H), 1.47 - 1.31 (m, 10H)

### 4-7. Synthesis of cyclohexyl 8-((2-hydroxyethyl)amino)octanoate

In a 250 mL 3-neck RBF, cyclohexyl 8-bromooctanoate (3.00 g, 9.83 mmol, 1.00 eq), 2-aminoethanol-1-ol (3.00 g, 49.1 mmol, 5.00 eq), Na₂CO₃ (1.04 g, 9.83 mmol, 1.00 eq) and 1,4-dioxane (60 mL) were added, and the mixture was purged three times with nitrogen, and stirred at 80 °C for 16 hours in a nitrogen environment. The reaction mixture was filtered, the filtrate was concentrated in vacuo, and the concentrated residue was purified by silica column chromatography (DCM:MeOH = 100:1 → 10:1) to give cyclohexyl 8-((2-hydroxyethyl)amino)octanoate (2.80 g, 9.81 mmol, 99.8% yield) as a yellow oil.
¹H NMR (400 MHz, CHLOROFORM-*d*): *δ* 4.77 (td, 1H), 3.75 - 3.67 (m, 2H), 2.91 - 2.80 (m, 5H), 2.68 (t, 2H), 2.29 (t, 2H), 1.88 - 1.82 (m, 2H), 1.77 - 1.70 (m, 2H), 1.66 - 1.60 (m, 2H), 1.55(br d, 2H), 1.46 - 1.28 (m, 12H)

### 4-8. Synthesis of the compound of formula D

In a 50 mL 3-neck RBF, cyclohexyl 8-((2-hydroxyethyl)amino)octanoate (942 mg, 3.30 mmol, 1.10 eq) and EtOH (10 mL) were added, and (1R,2R)-2-pentylcyclopropyl 8-bromooctanoate (1.00 g, 3.00 mmol, 1.00 eq) and DIEA (1.94 g, 15.00 mmol, 5.00 eq) were added thereto, and the mixture was stirred at 90 °C for 16 hours in a nitrogen environment. The reaction mixture was filtered, the filtrate was concentrated in vacuo, and the concentrated residue was purified by silica column chromatography (DCM:MeOH = 10:1 → 1:100) to obtain the compound of formula D (0.15 g, 279 µmol, 9.30% yield) as a yellow oil.
¹H NMR (400 MHz, CHLOROFORM-*d*): *δ* 5.23 - 4.94 (m, 2H), 4.63 - 4.56 (m, 1H), 4.12 (br s, 2H), 3.68 - 3.57 (m, 2H), 3.56 - 3.37 (m, 4H), 2.40 - 2.27 (m, 2H), 2.04 (br d, 6H), 1.91 - 1.76 (m, 2H), 1.74 - 1.55 (m, 17H), 1.54 - 1.46 (m, 2H), 1.43 - 1.25 (m, 5H), 1.21 (s, 2H), 0.90 (d, 6H)

### Example 5

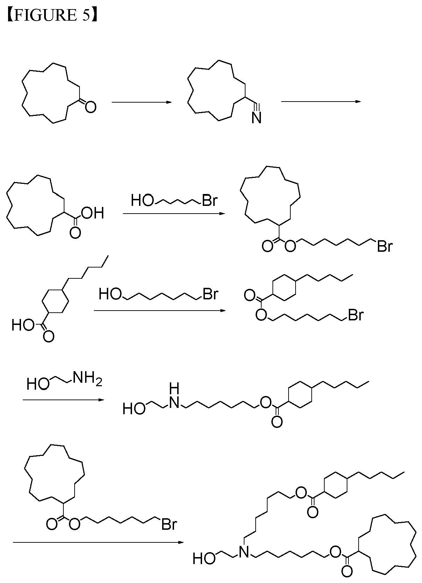

### 5-1. The compound of the following formula E was prepared according to the synthesis scheme shown in Figure 5.

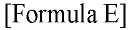

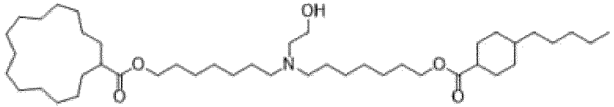

### 5-2. Synthesis of cyclopentadecanecarbonitrile

In a 2000 mL 3-neck RBF, cyclopentadecanone (25.0 g, 111 mmol, 1.00 eq), potassium, 2-methylpropan-2-olate (25.0 g, 223 mmol, 2.00 eq) and 2-methylpropan-2-ol (250 mL) were added together with THF (500 mL), and the mixture was cooled to 0 °C, and then 1-(isocyanomethylsulfonyl)-4-methyl-benzene (32.6 g, 167 mmol, 1.50 eq) was slowly added thereto at 0 °C over 1 hour, and the mixture was stirred at 20 °C for 11 hours. The reaction mixture was diluted with H₂O (400 mL) and extracted with EtOAc (400 mL), and the organic layer was washed with H₂O (400 mL), dried over Na₂SO₄ and filtered, and the filtrate was concentrated in vacuo, and the concentrated residue was purified by silica column chromatography (petroleum ether:EtOAc = 1:0 → 5:1) to give cyclopentadecanecarbonitrile (20 g, 84.96 mmol, 76.25% yield) as a colorless oil.
¹H NMR (400 MHz, CHLOROFORM-*d*): *δ* 1.33 (br s, 20 H) 1.44 - 1.53 (m, 4 H) 1.67 (q, 4 H) 2.59 (quin, 1 H)

### 5-3. Synthesis of cyclopentadecanecarboxylic acid

In a 250 mL 3-neck RBF, cyclopentadecanecarbonitrile (14.0 g, 59.5 mmol, 1.00 eq), KOH (6.00 M, 69.4 mL, 7.00 eq) and EtOH (70 mL) were added, and the mixture was purged three times with nitrogen, and stirred at 100 °C for 16 hours in a nitrogen environment. The reaction mixture was concentrated in vacuo, and the concentrated residue was acidified to pH 4 with 4 M aqueous hydrochloric acid solution (50 mL), and extracted with EtOAc (50 mL x 3). The organic layers were collected, dried over Na₂SO₄ and filtered, and the filtrate was concentrated in vacuo. The concentrated residue was purified by silica column chromatography (petroleum ether:EtOAc = 10:1 → 1:1) to give cyclopentadecanecarboxylic acid (1.20 g, 4.72 mmol, 7.93% yield) as a white solid.
¹H NMR (400 MHz, CHLOROFORM-*d*): *δ* ppm 1.27 - 1.46 (m, 24 H) 1.55 - 1.72 (m, 4 H) 2.44 (quin, 1 H) 10.15 - 11.75 (m, 1 H)

### 5-4. Synthesis of 7-bromoheptyl cyclopentadecanecarboxylate

In a 50 mL 3-neck RBF, cyclopentadecanecarboxylic acid (1.20 g, 4.72 mmol, 1.10 eq) and toluene (12 mL) were added, and 7-bromoheptan-1-ol (837 mg, 4.29 mmol, 1.00 eq) was added thereto, followed by H₂SO₄ (84.1 mg, 858 µmol, 45.7 µL, 0.20 eq), and the mixture was purged three times with nitrogen, and stirred at 120 °C for 16 hours in a nitrogen environment. The reaction mixture was concentrated in vacuo, and the concentrated residue was purified by silica column chromatography (petroleum ether:EtOAc = 10:1) to give 7-bromoheptyl cyclopentadecanecarboxylate (0.90 g, 2.09 mmol, 48.6% yield) as a colorless oil.
¹H NMR (400 MHz, CHLOROFORM-*d*): *δ* 1.29 - 1.40 (m, 30 H) 1.56 - 1.64 (m, 6 H) 1.87 (quin2 H) 2.34 - 2.46 (m, 1 H) 3.41 (t, 2 H) 4.07 (t, 2 H)

### 5-5. Synthesis of 7-bromoheptyl 4-pentylcyclohexane-1-carboxylate

In a 250 mL 3-neck RBF, 4-pentylcyclohexane-1-carboxylic acid (4.47 g, 22.9 mmol, 1.00 eq) and toluene (50 mL) were added, and 7-bromoheptan-1-ol (5.00 g, 25.2 mmol, 1.00 eq) was added thereto, followed by H₂SO₄ (450 mg, 4.58 mmol, 244 µL, 0.20 eq), and the mixture was purged three times with nitrogen, and stirred at 120 °C for 16 hours in a nitrogen environment. The reaction mixture was concentrated in vacuo, and the concentrated residue was purified by silica column chromatography (petroleum ether:EtOAc = 10:1) to give 7-bromoheptyl 4-pentylcyclohexane-1-carboxylate (7.00 g, 18.7 mmol, 81.4% yield) as a colorless oil.
¹H NMR (400 MHz, CHLOROFORM-*d*): *δ* 0.86 - 0.90 (m, 3 H) 1.18 - 1.32 (m, 10 H) 1.32 - 1.40 (m, 5 H) 1.41 - 1.59 (m, 6 H) 1.59 - 1.68 (m, 2 H) 1.81 - 1.90 (m, 2 H) 1.91 - 2.02 (m, 2 H) 2.46 - 2.54 (m, 1 H) 3.41 (t, 2 H) 4.02 - 4.12 (m, 2 H)

### 5-6. Synthesis of 7-((2-hydroxyethyl)amino)heptyl 4-pentylcyclohexane-1-carboxylate

In a 250 mL 3-neck RBF, 7-bromoheptyl 4-pentylcyclohexane-1-carboxylate (3.00 g, 7.99 mmol, 1.00 eq), 2-aminoethanol-1-ol (2.44 g, 40.0 mmol, 2.41 mL, 5.00 eq) and 1,4-dioxane (90 mL) were added, and the mixture was purged three times with nitrogen, and stirred at 100 °C for 16 hours in a nitrogen environment. The reaction mixture was concentrated in vacuo, and the concentrated residue was purified by silica column chromatography (DCM:MeOH = 10:1) to give 7-((2-hydroxyethyl)amino)heptyl 4-pentylcyclohexane-1-carboxylate (1.70 g, 4.78 mmol, 59.8% yield) as a yellow oil.
¹H NMR (400 MHz, CHLOROFORM-*d*): *δ 0.88* (t, 3 H) 1.19 - 1.28 (m, 8 H) 1.34 (br s, 6 H) 1.41 - 1.71 (m, 8 H) 1.84 - 2.01 (m, 6 H) 2.42 - 2.54 (m, 1 H) 2.63 (t, 2 H) 2.74 - 2.86 (m, 2 H) 3.50 - 3.79 (m, 2 H) 3.99 - 4.13 (m, 2 H)

### 5-7. Synthesis of the compound of formula E

In a 50 mL 3-neck RBF, 7-((2-hydroxyethyl)amino)heptyl 4-pentylcyclohexane-1-carboxylate (90 mg, 209 µmol, 1.00 eq) and 1,4-dioxane (5 mL) were added, and 7-bromoheptyl cyclopentadecanecarboxylate (74.2 mg, 209 µmol, 1.00 eq) and DIEA (135 mg, 1.04 mmol, 5.00 eq) were added thereto, and the mixture was purged three times with nitrogen, and stirred at 105 °C for 16 hours in a nitrogen environment. The reaction mixture was cooled to 25 °C, water (10 mL) was added to quench the reaction, and the mixture was extracted with 30 mL of EtOAc (10 mL x 3). The organic layers were collected, dried over Na₂SO₄ and filtered, and the filtrate was concentrated in vacuo. The concentrated residue was purified by silica column chromatography (DCM:MeOH = 10:1) to obtain the compound of formula E (70 mg, 99.1 µmol, 47.5% yield) as a yellow oil.
¹H NMR (400 MHz, CHLOROFORM-*d*): *δ* 0.88 (t, 3 H) 1.23 - 1.40 (m, 46 H) 1.45 - 1.50 (m, 4 H) 1.52 - 1.66 (m, 12 H) 1.72 - 2.05 (m, 4 H) 2.37 - 2.55 (m, 6 H) 2.63 (br t, 2 H) 3.57 (br t, 2 H) 4.02 - 4.09 (m, 4 H)

### Example 6

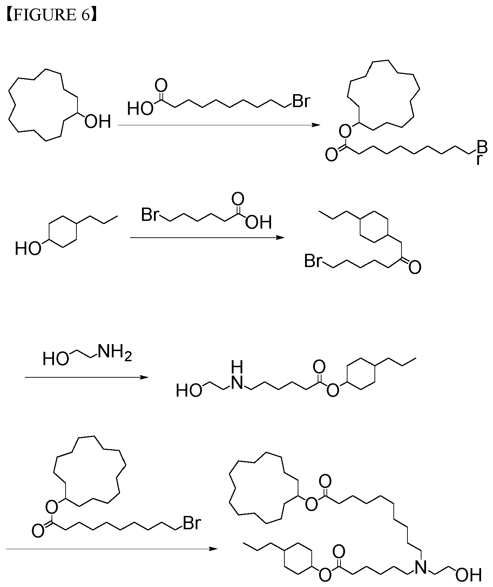

### 6-1. The compound of the following formula F was prepared according to the synthesis scheme shown in Figure 6.

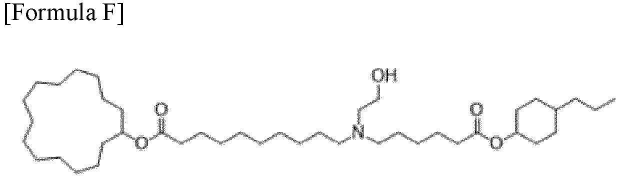

### 6-2. Synthesis of cyclopentadecyl 10-bromodecanoate

Synthesis was performed by the method described in Example 3-2.

### 6-3. Synthesis of 4-propylcyclohexyl 6-bromohexanoate

Synthesis was performed by the method described in Example 2-3.

### 6-4. Synthesis of 4-propylcyclohexyl 6-((2-hydroxyethyl)amino)hexanoate

Synthesis was performed by the method described in Example 2-4.

### 6-5. Synthesis of the compound of formula F

In a 50 mL 3-neck RBF, 4-propylcyclohexyl 6-((2-hydroxyethyl)amino)hexanoate (0.10 g, 1.00 eq), cyclopentadecyl 10-bromodecanoate (153 mg, 1.00 eq) and DIEA (44 mg, 1.00 eq) were added together with EtOH (5 mL), and the mixture was purged three times with nitrogen, and stirred at 95 °C for 16 hours in a nitrogen environment. The reaction mixture was cooled to room temperature and concentrated in vacuo, and the concentrated residue was purified by silica column chromatography (DCM:MeOH = 100:1 → 10:1) to give the compound of formula F (70 mg, 30.9% yield) as a yellow oil.
¹H NMR (400 MHz, CHLOROFORM-*d*): *δ* 5.03 - 4.62 (m, 2H), 4.01 - 3.72 (m, 2H), 3.10 - 2.75 (m, 6H), 2.35 - 2.25 (m, 4H), 1.95 (br d, 1H), 1.59 (br s, 18H), 1.40 - 1.17 (m, 42H), 1.05 - 0.96 (m, 1H), 0.92 - 0.87 (m, 3H)

### Example 7

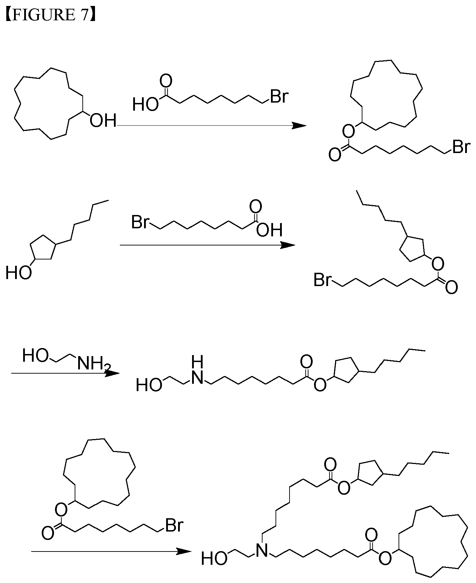

The compound of the following formula G was prepared according to the synthesis scheme shown in Figure 7 in the same manner as in Example 1, except that 3-pentylcyclopentan-1-ol was used instead of 4-pentylcyclohexan-1-ol. 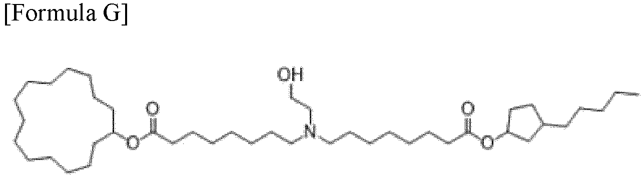 ¹H NMR (400 MHz, CHLOROFORM-*d*): *δ* 5.03 - 4.77 (m, 2H), 4.09 - 3.88 (m, 2H), 3.04 - 2.88 (br, 6H), 2.41 - 2.31 (m, 4H), 1.89 - 1.56 (br, 14H), 1.51 - 0.96 (br, 53H), 0.88 (t, 3H)

### Example 8

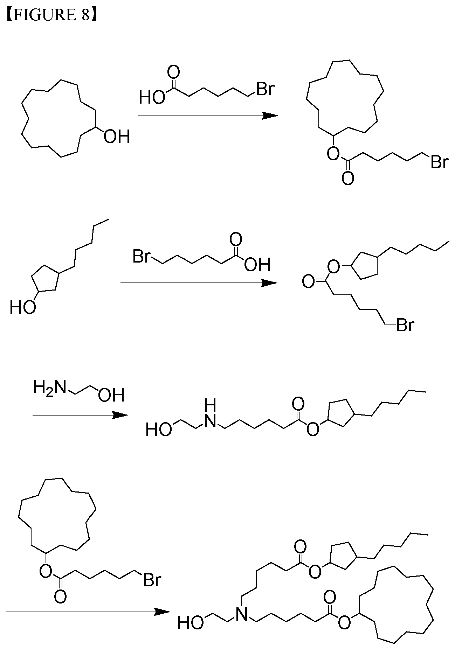

The compound of the following formula H was prepared according to the synthesis scheme shown in Figure 8 in the same manner as in Example 1, except that 6-bromohexanoic acid was used instead of 8-bromooctanoic acid and 3-pentylcyclopentan-1-ol was used instead of 4-pentylcyclohexan-1-ol. 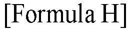 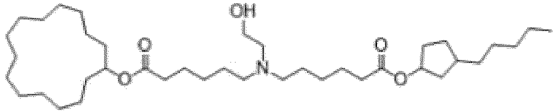 ¹H NMR (400 MHz, CHLOROFORM-*d*): *δ* 4.68 (m, 1H), 4.48 (m, 1H), 3.91 (m, 2H), 3.02 - 2.89 (br, 6H), 2.35 - 2.29 (m, 4H), 1.92 - 1.61 (m, 14H), 1.58 - 1.01 (m, 45H), 0.90 (t, 3H)

### Example 9

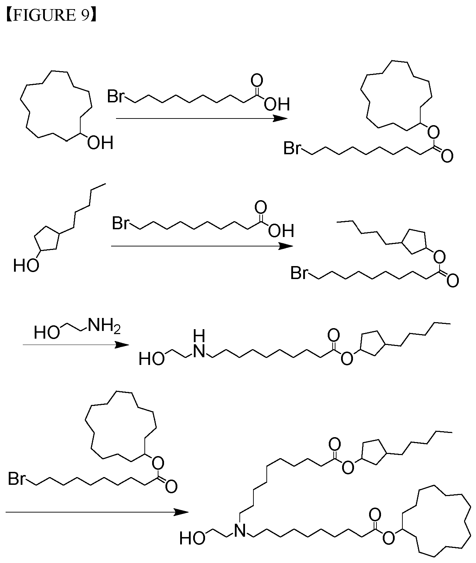

The compound of the following formula I was prepared according to the synthesis scheme shown in Figure 9 in the same manner as in Example 1, except that 10-bromodecanoic acid was used instead of 8-bromooctanoic acid and 3-pentylcyclopentan-1-ol was used instead of 4-pentylcyclohexan-1-ol. 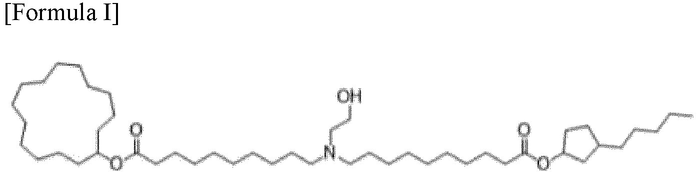 ¹H NMR (400 MHz, CHLOROFORM-*d*): *δ* 4.70 (m, 1H), 4.47 (m, 1H), 3.90 (m, 2H), 2.99 - 2.79 (br, 6H), 2.31 - 2.26 (m, 4H), 1.92 - 1.61 (m, 14H), 1.58 - 1.01 (m, 61H), 0.85 (t, 3H)

### Example 10

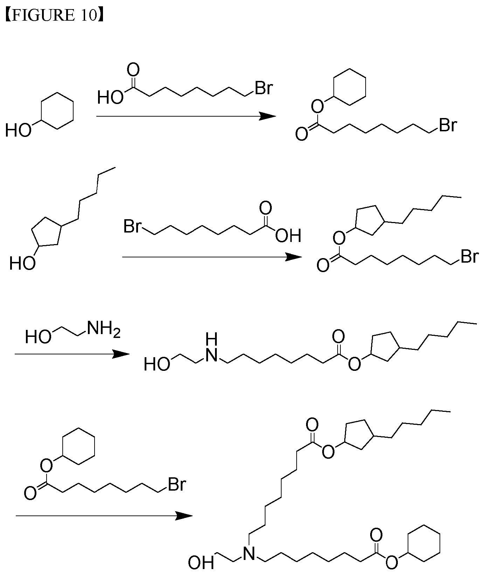

The compound of the following formula J was prepared according to the synthesis scheme shown in Figure 10 in the same manner as in Example 1, except that cyclohexanol was used instead of cyclopentadecanol and 3-pentylcyclopentan-1-ol was used instead of 4-pentylcyclohexan-1-ol. 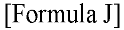 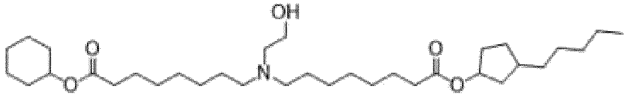 ¹H NMR (400 MHz, CHLOROFORM-*d*): *δ* 4.68 - 4.61 (m, 2H), 3.94 (m, 2H), 3.12 - 2.94 (br, 6H), 2.44 (m, 4H), 1.91 - 1.10 (m 45H), 0.88 (t, 3H)

### Example 11

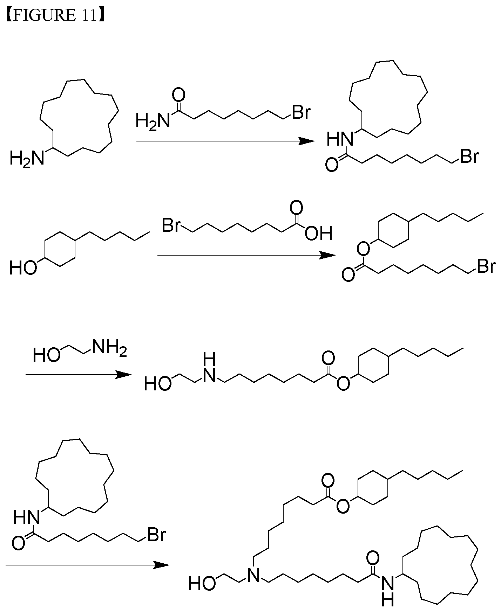

The compound of the following formula K was prepared according to the synthesis scheme shown in Figure 11 in the same manner as in Example 1, except that aminocyclopentadecane was used instead of cyclopentadecanol. 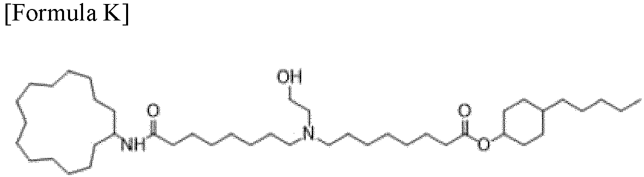 ¹H NMR (400 MHz, CHLOROFORM-*d*): *δ* 8.10 (br, 1H), 4.67 - 4.63 (m, 1H), 3.95 - 3.88 (m, 2H), 3.64 - 3.59 (m, 1H), 3.11 - 2.89 (br, 6H), 2.31 - 2.19 (m, 4H), 1.95 - 1.25 (m, 65H), 0.88 (t, 3H)

### Example 12

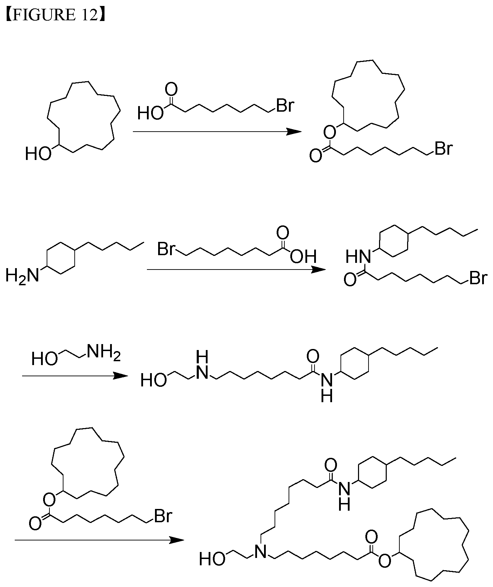

The compound of the following formula L was prepared according to the synthesis scheme shown in Figure 12 in the same manner as in Example 1, except that 1-amino-4-pentylcyclohexane was used instead of 4-pentylcyclohexan-1-ol. 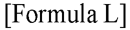 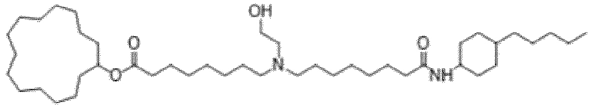 ¹H NMR (400 MHz, CHLOROFORM-*d*): *δ* 8.12 (br, 1H), 4.50 - 4.43 (m, 1H), 3.88 - 3.76 (m, 2H), 3.49 - 3.40 (m, 2H), 3.36 - 3.33 (m, 1H), 3.03 - 2.90 (br, 6H), 2.31 - 2.90 (m, 4H), 1.92 - 1.20 (m, 65H), 0.90 (t, 3H)

### Example 13

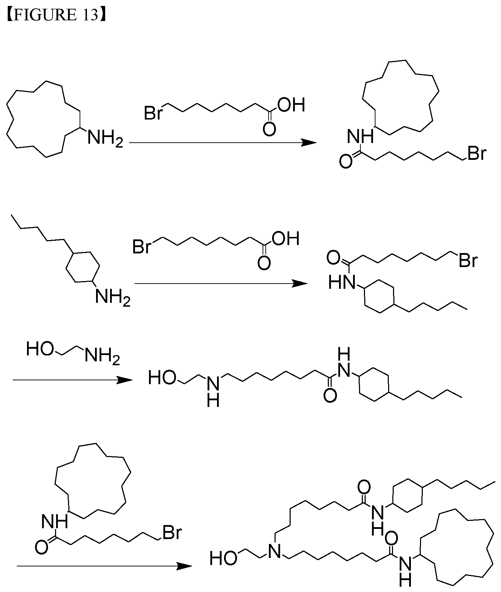

The compound of the following formula M was prepared according to the synthesis scheme shown in Figure 13 in the same manner as in Example 1, except that aminocyclopentadecane was used instead of cyclopentadecanol and 1-amino-4-pentylcyclohexane was used instead of 4-pentylcyclohexan-1-ol. 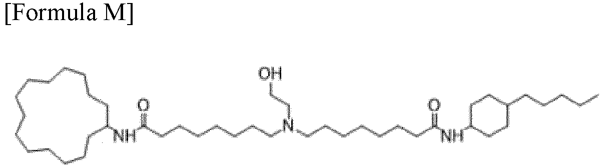 ¹H NMR (400 MHz, CHLOROFORM-*d*): *δ* 8.10 (br, 2H), 3.84 - 3.78 (m, 2H), 3.69 - 3.61 (m, 2H), 3.11 - 2.98 (br, 6H), 2.33 - 2.19 (m, 4H), 1.90 - 1.22 (m, 65H), 0.91 (t, 3H)

### Example 14

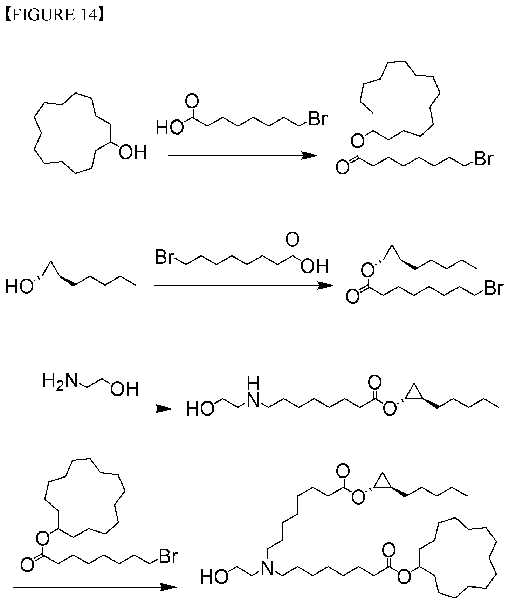

The compound of the following formula N was prepared according to the synthesis scheme shown in Figure 14 in the same manner as in Example 1, except that (1R,2R)-2-pentylcyclopropan-1-ol prepared in Example 4-4 was used instead of 4-pentylcyclohexan-1-ol. 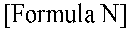 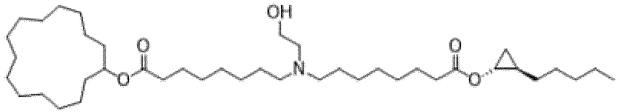 ¹H NMR (400 MHz, CHLOROFORM-*d*): *δ* 4.56 - 4.45 (m, 1H), 3.56 - 3.52 (m, 2H), 3.42 (br, 1H), 3.11 - 3.05 (br, 4H), 2.77 - 2.70 (br, 2H), 2.44 - 2.38 (m, 4H), 1.91 - 1.21 (m, 57H), 0.9 (t, 3H), 0.62 - 0.55 (m, 2H)

### Example 15

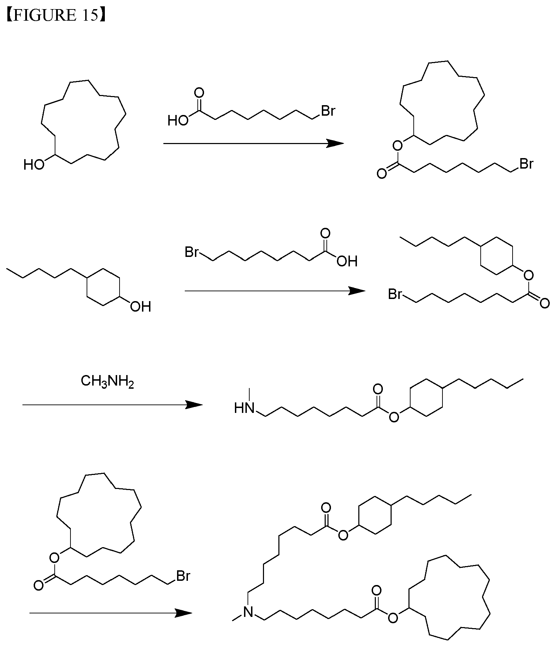

The compound of the following formula O was prepared according to the synthesis scheme shown in Figure 15 in the same manner as in Example 1, except that methylamine (CH₃-NH₂) was used instead of 2-aminoethanol-1-ol. 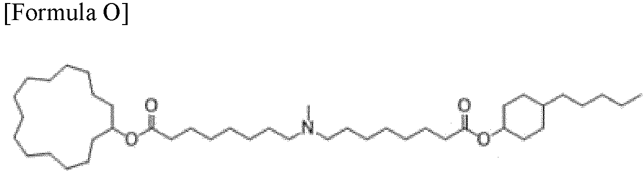 ¹H NMR (400 MHz, CHLOROFORM-*d*): *δ* 4.41 - 4.38 (m, 1H), 4.22 - 4.18 (m, 1H), 3.00 - 2.82 (m, 4H), 2.75 (s, 3H), 2.42 - 2.39 (m, 4H), 1.92 - 1.20 (m, 65H), 0.90 (t, 3H)

### Example 16

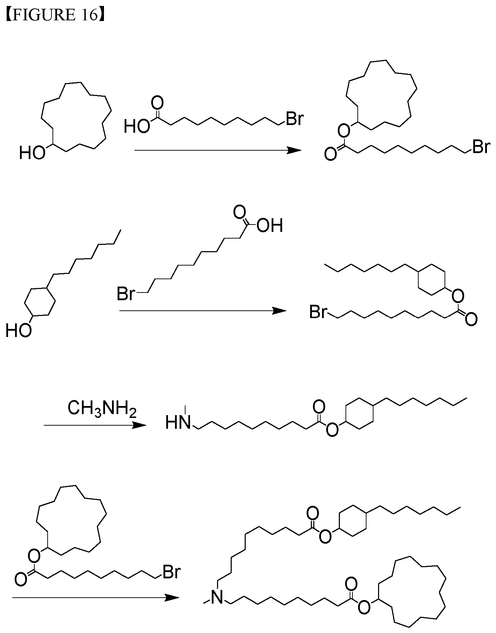

The compound of the following formula P was prepared according to the synthesis scheme shown in Figure 16 in the same manner as in Example 1, except that methylamine (CH₃-NH₂) was used instead of 2-aminoethanol-1-ol, 10-bromodecanoic acid was used instead of 8-bromooctanoic acid, and 4-heptylcyclohexan-1-ol was used instead of 4-pentylcyclohexan-1-ol. 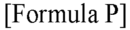 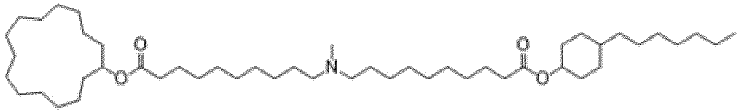 ¹H NMR (400 MHz, CHLOROFORM-*d*): *δ* 4.43 - 4.40 (m, 1H), 4.21 - 4.18 (m, 1H), 3.12 - 2.92 (m, 4H), 2.80 (s, 3H), 2.42 - 2.39 (m, 4H), 1.93 - 1.19 (m, 73H), 0.90 (t, 3H)

### Example 17

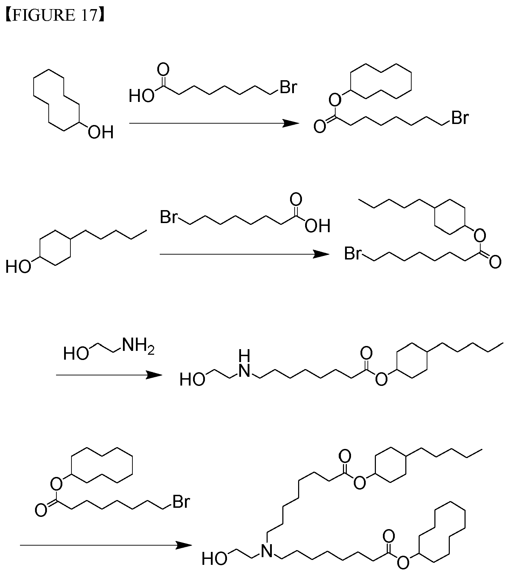

The compound of the following formula Q was prepared according to the synthesis scheme shown in Figure 17 in the same manner as in Example 1, except that cyclodecanol was used instead of cyclopentadecanol. 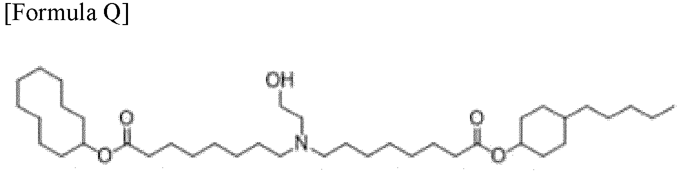 ¹H NMR (400 MHz, CHLOROFORM-*d*): *δ* 4.48 - 4.41 (m, 1H), 4.19 - 4.17 (m, 1H), 3.54 - 3.49 (m, 2H), 3.02 - 2.92 (br, 4H), 2.78 - 2.71 (br, 2H), 2.35 - 2.31 (m, 4H), 1.91 - 1.19 (m, 55H), 0.88 (t, 3H)

### Example 18

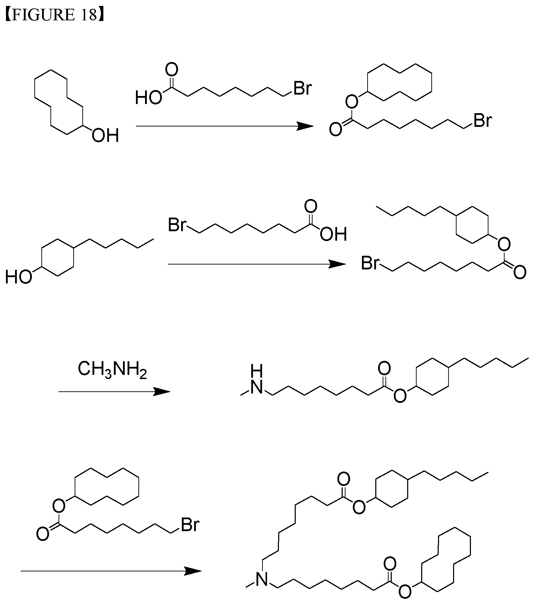

The compound of the following formula R was prepared according to the synthesis scheme shown in Figure 18 in the same manner as in Example 1, except that cyclodecanol was used instead of cyclopentadecanol and methylamine (CH₃-NH₂) was used instead of 2-aminoethanol-1-ol. 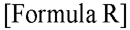 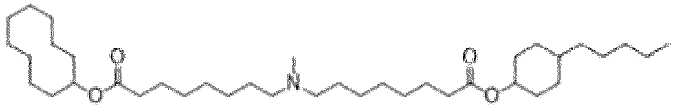 ¹H NMR (400 MHz, CHLOROFORM-*d*): *δ* 4.48 - 4.41 (m, 1H), 4.19 - 4.17 (m, 1H), 3.01 - 2.93 (br, 4H), 2.75 (br, 3H), 2.34 - 2.30 (m, 4H), 1.90 - 1.21 (m, 55H), 0.90 (t, 3H)

### [Preparation Examples of composition for drug delivery]

### 1. Raw material preparation

According to the following table, the materials required for preparing the formulation were dissolved in each dilution solvent and prepared at the required concentration. When dissolving, the materials were kept at room temperature and then the solvent was added to dissolve them.

| | **Materials** | **Concentration for use** |
|---|---|---|
| 1 | mRNA | 1 mg/mL |
| 2 | Each compound of formulas A to F | 20 mg/mL |
| 3 | DOPE (dioleoyl-phosphatidyl-ethanolamine) | 10 mg/mL |
| 4 | Cholesterol | 10 mg/mL |
| 5 | DMG-PEG (1,2-dimyristoyl-rac-glycero-3-methoxypolyethylene glycol-2000) | 10 mg/mL |

### 2. Raw material mixing

The required amounts of the raw materials were mixed in order to meet the N/P ratio (amine group of lipid/phosphate group of mRNA) of 6 and each compound of formulas A to F:DOPE:Cholesterol:DMG-PEG=50:10:38.5:1.5. Ethanol was added to the ethanol layer so that the molecular total of all raw materials was within 12.5 mM, and the aqueous phase and ethanol phase were mixed maintaining a volume ratio of 3:1. After the mixing, to reduce the total ethanol content, buffer exchange was performed as follows: The mixture solution was concentrated by centrifugation at 4,000 rpm to 1/3 level using an Amicon-Ultra tube filter (Merk Millipore, UFC505096 or UFC805024, pore size: 50K, volume: 15 mL), then diluted with three times the volume of PBS and centrifuged to concentrate. This process was repeated six times to exchange the buffer.

The more concrete procedure is as follows:
1) Two autoclaved tubes were prepared (tubes (A) and (B)).
2) In tube (A), each compound of formulas A to F, DOPE, cholesterol and DMG-PEG in molar quantities calculated according to the experimental conditions were sequentially added and mixed by vortexing.
3) In the ethanol phase, when necessary, ethanol was added so that the molecular total of all raw materials was within 12.5 mM.
4) In tube (B), mRNA and 20 mM sodium acetate buffer (pH 4.6) (=prepared by diluting with 3M sodium acetate buffer to 20 mM and titrating to pH 4.6 using 1M HCl) were mixed. At that time, the ratio was calculated and added so that the aqueous phase was total three times the amount of the ethanol phase.
5) Mixing of Tube (A) and Tube (B) was performed using a Microfluidics (Ignite, Precision Nanosystem) device. Microfluidics operating conditions were FRR (Flow Rate Ratio) of C:R=3:1 and TRR (Total Flow Rate) of 12 mL/min.
6) The resulting mixture from step 5) was concentrated by centrifugation at 4,000 rpm to 1/3 level using an Amicon-Ultra tube filter (50K), then diluted 3-fold with PBS and concentrated by centrifugation. This process was repeated 6 times for concentration to the desired volume.

### 3. Evaluation of the properties of the formulation

1) For the prepared formulation, particle characteristics (i.e., Zeta-average particle size (Z-average), polydispersity index (PDI), and zeta-potential) were confirmed using a particle size analyzer (Dynamic Light Scattering, DLS), and the results are shown in Table 1 below.
2) For the prepared formulation, mRNA encapsulation efficiency was confirmed through Ribo-green assay, and the results are shown in Table 1 below.

**[Table 1]**

| **Lipid compound** | **Z-average (nm)** | **PDI (PI)** | **Zeta-potential (mV)** | **Encapsulation efficiency (%)** |
|---|---|---|---|---|
| Example 1 | 129.98 | 0.07 | -0.84 | 94.0 |
| Example 2 | 153.5 | 0.16 | -7.54 | 99.7 |
| Example 3 | 144.53 | 0.14 | -3.36 | 99.0 |
| Example 4 | 173.17 | 0.12 | -10.81 | 96.7 |
| Example 5 | 121.1 | 0.09 | -3.36 | 99.9 |
| Example 6 | 155.03 | 0.08 | -5.80 | 99.7 |

## Claims

1. A a lipid having a structure represented by the following formula (1): 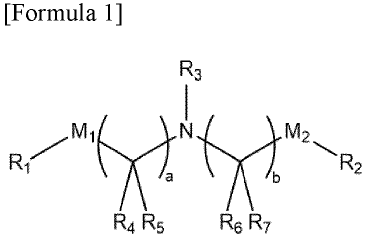
wherein, in the above formula (1),
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₇ is independently hydrogen atom, or a substituted or unsubstituted, saturated or unsaturated hydrocarbon group, and
each of a and b is independently an integer of from 1 to 20.

2. The lipid according to claim 1, wherein
each of M₁ and M₂ is independently selected from the group consisting of -C(O)O-, - OC(O)-, -OC(O)-M'-C(O)O-, -C(O)N(R')-, -N(R')C(O)-, -C(O)-, -C(S)-, -C(S)S-, - SC(S)-, -CH(OH)-, -P(O)(OR')O-, -S(O)₂-, -S-S-, arylene (more concretely, C₆₋₂₀ arylene), and heteroarylene (more concretely, C₃₋₂₀ heteroarylene having one or more (e.g., 1 to 3) heteroatoms selected from N, O and S), wherein M' is a direct bond, C₁₋₁₃ alkylene or C₂₋₁₃ alkenylene, and each R' is independently selected from the group consisting of hydrogen atom, C₁₋₁₈ alkyl and C₂₋₁₈ alkenyl,
each of R₁ and R₂ is independently selected from the group consisting of C₃₋₂₀ cycloalkyl, C₃₋₂₀ cycloalkenyl, C₆₋₂₀ aryl, C₃₋₂₀ heterocycloalkyl, C₃₋₂₀ heterocycloalkenyl, and C₃₋₂₀ heteroaryl, each of which is independently unsubstituted or substituted with C₁₋₁₈ alkyl or C₂₋₁₈ alkenyl,
R₃ is selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, - CHQR and -CQ(R)₂, wherein each R is independently selected from the group consisting of hydrogen atom, C₁₋₃ alkyl and C₂₋₃ alkenyl; Q is selected from the group consisting of carbocyclic group, heterocyclic group, -OR, -O(CH₂)ₙN(R)₂, -C(O)OR, -OC(O)R, -CX₃, - CX₂H, -CXH₂, -CN, -N(R)₂, -C(O)N(R)₂, -N(R)C(O)R, -N(R)S(O)₂R, -N(R)C(O)N(R)₂, - N(R)C(S)N(R)₂, -N(R)R₁₂, N(R)S(O)2R₁₂, -O(CH2)ₙOR, -N(R)C(=NR₁₃)N(R)₂, - N(R)C(=CHR₁₃)N(R)₂, -OC(O)N(R)₂, -N(R)C(O)OR, -N(OR)C(O)R, -N(OR)S(O)₂R, - N(OR)C(O)OR, -N(OR)C(O)N(R)₂, -N(OR)C(S)N(R)₂, -N(OR)C(=NR₁₃)N(R)₂, - N(OR)C(=CHR₁₃)N(R)₂, -C(=NR₁₃)N(R)₂, - C(=NR₁₃)R, -C(O)N(R)OR and - C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, -S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is -(CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl,
each of R₄ to R₇ is independently selected from the group consisting of hydrogen atom, C₁₋₃ alkyl and C₂₋₃ alkenyl, and
each of a and b is independently an integer of from 1 to 15.

3. The lipid according to claim 2, wherein
each of M₁ and M₂ is independently selected from the group consisting of -C(O)O-, - OC(O)-, -OC(O)-M'-C(O)O-, -C(O)N(R')-, -N(R')C(O)- and -C(O)-, wherein M' and R' are the same as defined in claim 2,
each of R₁ and R₂ is independently selected from the group consisting of C₃₋₂₀ cycloalkyl and C₃₋₂₀ heterocycloalkyl, each of which is independently unsubstituted or substituted with C₁₋₁₈ alkyl or C₂₋₁₈ alkenyl,
R₃ is selected from the group consisting of hydrogen atom, substituted or unsubstituted C₁₋₆ alkyl, and substituted or unsubstituted C₃₋₆ carbocyclic group,
each of R₄ to R₇ is independently hydrogen atom or C₁₋₃ alkyl, and
each of a and b is independently an integer of from 3 to 13.

4. The lipid according to claim 3, wherein
each of M₁ and M₂ is independently selected from the group consisting of -C(O)O-, - OC(O)-, -C(O)N(R')- and -N(R')C(O)-, wherein each R' is independently selected from the group consisting of hydrogen atom, C₁₋₁₈ alkyl and C₂₋₁₈ alkenyl,
each of R₁ and R₂ is independently substituted or unsubstituted C₃₋₁₅ cycloalkyl, R₃ is hydrogen atom, or substituted or unsubstituted C₁₋₃ alkyl,
R₄ to R₇ are hydrogen atom, and
each of a and b is independently an integer of from 5 to 11.

5. The lipid according to claim 4, wherein the lipid is one having a structure selected from the following formulas A to R:
| Formula | Structure | Formula | Structure |
|---|---|---|---|
| A | 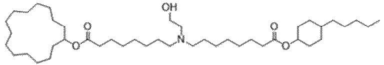 | J | 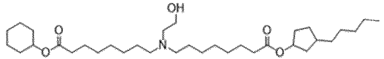 |
| B | 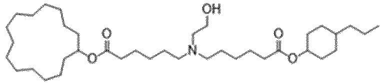 | K | 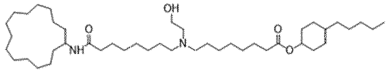 |
| C | 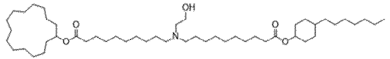 | L | 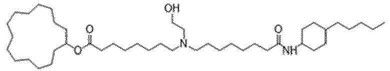 |
| D | 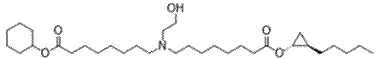 | M | 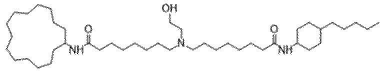 |
| E | 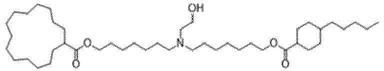 | N | 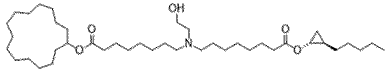 |
| F | 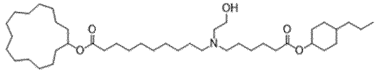 | O | 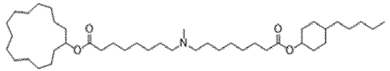 |
| G | 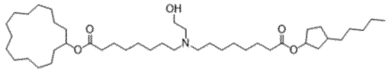 | P | 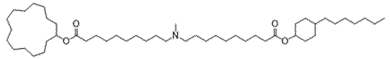 |
| H | 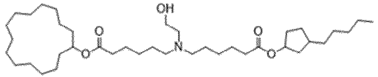 | Q | 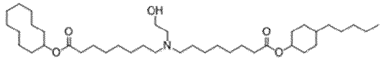 |
| I | 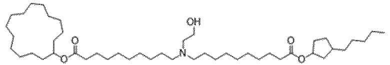 | R | 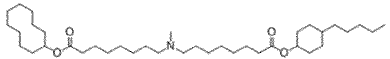 |

6. A method for preparing a lipid having a structure represented by formula (1'), comprising steps of:
(1) reacting a compound of formula (a) with a compound of formula (b) to obtain a compound of formula (c); and
(2) reacting a compound of formula (c) with a compound of formula (d):
[Formula a] R₁-OH 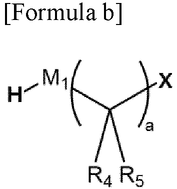 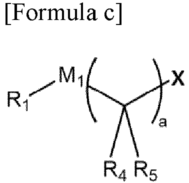
[Formula d] R₃-NH₂ 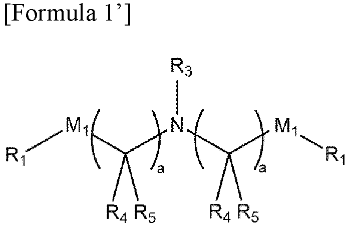
wherein,
M₁, R₁, R₃, R₄, R₅ and a are the same as defined in claim 1, and
each X is independently selected from the group consisting of F, CI, Br and I.

7. A method for preparing a lipid having a structure represented by formula (1), comprising steps of:
(1) reacting a compound of formula (a') with a compound of formula (b') to obtain a compound of formula (c');
(2) reacting a compound of formula (c') with a compound of formula (d) to obtain a compound of formula (e); and
(3) reacting a compound of formula (e) with a compound of formula (c) obtained in claim 6:
[Formula a'] HO-R₂ 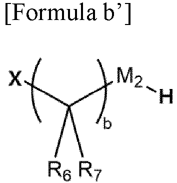 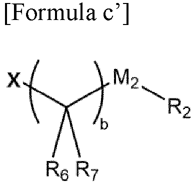
[Formula d] R₃-NH₂ 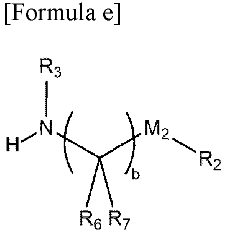 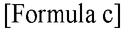 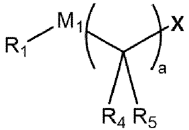 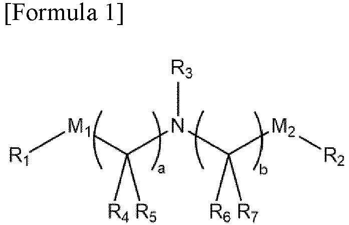
wherein,
M₁, M₂, R₁ to R₇, a and b are the same as defined in claim 1, and
each X is independently selected from the group consisting of F, CI, Br and I.

8. A method for preparing a lipid having a structure represented by formula (1'), comprising steps of:
(1) reacting a compound of formula (i) with a compound of formula (ii) to obtain a compound of formula (iii); and
(2) reacting a compound of formula (iii) with a compound of formula (iv):
[Formula i] R₁-M₁-H 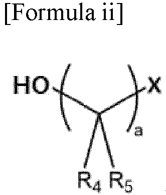 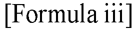 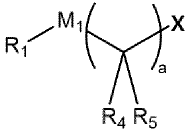
**[Formula** iv] R₃-NH₂ 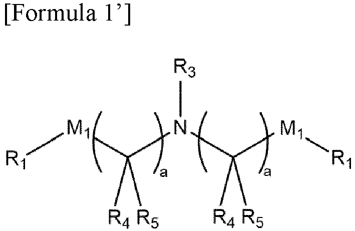
wherein,
M₁, R₁, R₃, R₄, R₅ and a are the same as defined in claim 1, and
each X is independently selected from the group consisting of F, CI, Br and I.

9. A method for preparing a lipid having a structure represented by formula (1), comprising steps of:
(1) reacting a compound of formula (i') with a compound of formula (ii') to obtain a compound of formula (iii');
(2) reacting a compound of formula (iii') with a compound of formula (iv) to obtain a compound of formula (v); and
(3) reacting a compound of formula (v) with a compound of formula (iii) obtained in claim 8:
[Formula i'] H-M₂-R₂ 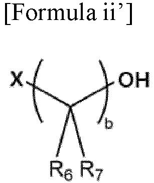 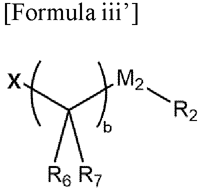
**[Formula** iv] R₃-NH₂ 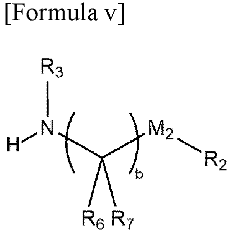 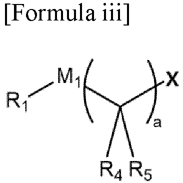 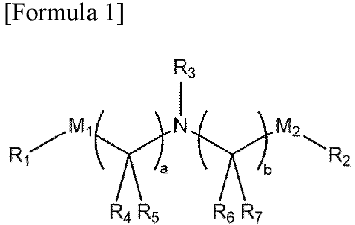
wherein,
M₁, M₂, R₁ to R₇, a and b are the same as defined in claim 1, and
each X is independently selected from the group consisting of F, CI, Br and I.

10. A composition for drug delivery comprising the lipid of any one of claims 1 to 5.
